# EUROPÄISCHE PATENTSCHRIFT

(11) **EP 2 059 600 B1**
(45) Veröffentlichungstag und Bekanntmachung des Hinweises auf die Patenterteilung: **02.04.2014**
(21) Anmeldenummer: 07802807.3
(22) Anmeldetag: 22.08.2007
(51) Int. Cl.: C12N 15/82

(54) **VERFAHREN ZUR ERHÖHUNG DER PATHOGENRESISTENZ IN TRANSGENEN PFLANZEN**
METHOD FOR INCREASING RESISTANCE TO PATHOGENS IN TRANSGENIC PLANTS
PROCÉDÉ D'AUGMENTATION DE LA RÉSISTANCE AUX AGENTS PATHOGÈNES DES PLANTES TRANSGÉNIQUES

(30) Priorität: 30.08.2006 EP 06119815
(43) Veröffentlichungstag der Anmeldung: 20.05.2009
(73) Patentinhaber: BASF Plant Science GmbH, 67056 Ludwigshafen (DE)
(72) Erfinder: DOUCHKOV, Dimitar, 06466 Gatersleben (DE); SCHWEIZER, Patrick, 06493 Ballenstedt (DE)
(74) Vertreter: Piening, Niklas
(86) Internationale Anmeldenummer: PCT/EP2007/058735
(87) Internationale Veröffentlichungsnummer: WO 2008/025711

(56) Entgegenhaltungen:
- WO-A-99/32619
- BIERI STPHANE ET AL: "RAR1 positively controls steady state levels of barley MLA resistance proteins and enables sufficient MLA6 accumulation for effective resistance" PLANT CELL, Bd. 16, Nr. 12, Dezember 2004 (2004-12), Seiten 3480-3495, XP002468496 ISSN: 1040-4651
- SUN XINLI ET AL: "Xa26, a gene conferring resistance to Xanthomonas oryzae pv. oryzae in rice, encodes an LRR receptor kinase-like protein." PLANT JOURNAL, Bd. 37, Nr. 4, Februar 2004 (2004-02), Seiten 517-527, XP002468497 ISSN: 0960-7412
- DATABASE EMBL [Online] 26. Februar 2005 (2005-02-26), "HO30D02w HO Hordeum vulgare cDNA clone HO30D02 3-PRIME, mRNA sequence." XP002468498 gefunden im EBI accession no. EMBL:DN186179 Database accession no. DN186179 -& ZIEROLD UWE ET AL: "Transcriptome analysis of mlo-mediated resistance in the epidermis of barley" MOLECULAR PLANT PATHOLOGY, XX, XX, Bd. 6, Nr. 2, März 2005 (2005-03), Seiten 139-151, XP002425684 ISSN: 1464-6722
- DATABASE EMBL [Online] 26. Februar 2005 (2005-02-26), "HO28P17w HO Hordeum vulgare cDNA clone HO28P17 3-PRIME, mRNA sequence." XP002468499 gefunden im EBI accession no. EMBL:DN186648 Database accession no. DN186648 & ZIEROLD UWE ET AL: "Transcriptome analysis of mlo-mediated resistance in the epidermis of barley" MOLECULAR PLANT PATHOLOGY, XX, XX, Bd. 6, Nr. 2, März 2005 (2005-03), Seiten 139-151, XP002425684 ISSN: 1464-6722
- DOUCHKOV D ET AL: "A high-throughput gene-silencing system for the functional assessment of defense-related genes in barley epidermal cells" MOLECULAR PLANT-MICROBE INTERACTIONS, APS PRESS, ST. PAUL, MN, US, Bd. 18, Nr. 8, August 2005 (2005-08), Seiten 755-761, XP002397481 ISSN: 0894-0282
- SCHWEIZER P ET AL: "Double-stranded RNA interferes with gene function at the single-cell level in cereals" PLANT JOURNAL, BLACKWELL SCIENTIFIC PUBLICATIONS, OXFORD, GB, Bd. 24, Nr. 6, Dezember 2000 (2000-12), Seiten 895-903, XP002397482 ISSN: 0960-7412
- ROST B: "Enzyme Function Less Conserved than Anticipated", JOURNAL OF MOLECULAR BIOLOGY, LONDON, GB LNKD- DOI:10.1016/S0022-2836(02)00016-5, vol. 318, no. 2, 26 April 2002 (2002-04-26), pages 595-608, XP004449547, ISSN: 0022-2836

## Beschreibung

Die vorliegende Erfindung betrifft ein Verfahren zur Erhöhung der Pathogenresistenz in transgenen Pflanzen und/oder Pflanzenzellen, wobei eine DNA-Sequenz, die für ein Protein mit einer leucine-rich-repeat (LRR)-Domäne und/oder einer Kinaseaktivität kodiert, in die Pflanze bzw. Pflanzenzelle eingeführt und dort exprimiert wird. Ebenfalls offenbart ist die Verwendung von Nukleinsäuren, die für ein solches Protein kodieren, zur Herstellung von transgenen Pflanzen bzw. Pflanzenzellen mit einer erhöhten Pathogenresistenz. Weiterhin offenbart sind Nukleinsäuresequenzen, die für ein Protein kodieren, das eine erhöhte Pathogenresistenz in Pflanzen vermittelt.

Pflanzenkrankheiten, die durch verschiedene Pathogene wie z.B. Viren, Bakterien und Pilzen verursacht werden, können beim Anbau von Kulturpflanzen zu erheblichen Ernteausfällen führen, was zum einen wirtschaftliche Folgen hat, aber auch die Sicherheit der menschlichen Ernährung bedroht. Seit dem letzten Jahrhundert werden zur Kontrolle von Pilzerkrankungen chemische Fungizide eingesetzt. Durch den Einsatz dieser Stoffe konnte zwar das Ausmaß von Pflanzenerkrankungen reduziert werden, es ist allerdings bis heute nicht auszuschließen, dass diese Verbindungen eine schädliche Wirkung auf Mensch, Tier und Umwelt ausüben. Um langfristig den Verbrauch von herkömmlichen Pflanzenschutzmitteln auf ein Minimum zu reduzieren, ist es daher von Bedeutung, die natürliche Pathogenabwehr von verschiedenen Pflanzen gegenüber unterschiedlichen Erregern zu untersuchen und sie sich durch gentechnologische Manipulation, z.B. durch das Einführen externer Resistenzgene oder durch die Manipulation der endogenen Genexpression in den Pflanzen, zur Herstellung von pathogenresistenten Pflanzen gezielt nutzbar zu machen.

Es gibt nur wenige Ansätze, die Pflanzen eine Resistenz gegenüber Pathogenen, vor allem Pilzpathogenen, verleihen. Dieser Mangel ist zum Teil in der Komplexität der betrachteten biologischen Systeme begründet. Dem Erreichen von Resistenzen gegenüber Pathogenen steht auch entgegen, dass über die Wechselwirkungen zwischen Pathogen und Pflanze wenig bekannt ist. Die große Anzahl unterschiedlicher Pathogene, die von ihnen entwickelten Infektionsmechanismen und die von den Pflanzenstämmen, Familien und Arten entwickelten Abwehrmechanismen stehen in vielfältigen Wechselwirkungen miteinander.

Pilzpathogene haben im Wesentlichen zwei Infektionsstrategien entwickelt. Manche Pilze dringen über die Spaltöffnungen in das Wirtsgewebe ein (z.B. Rostpilze, Septoria-, Fusarium-Arten) und penetrieren das Mesophyllgewebe, während andere über die Cuticula die darunter liegenden Epidermiszellen penetrieren (z.B. Blumeria-Arten).

Die durch die Pilzpathogene verursachten Infektionen führen in den befallenen Pflanzen zur Aktivierung der pflanzlichen Abwehrmechanismen. So konnte gezeigt werden, dass Abwehrreaktionen gegen Epidermis-penetrierende Pilze häufig mit der Ausbildung einer Penetrationsresistenz (Papillenbildung, Zellwandverdickungen mit Kallose als Hauptbestandteil) unterhalb der pilzlichen Penetrationshyphe beginnen (Elliott et al. (2002) Mol. Plant Microbe Interact. 15: 1069-77).

Die pflanzlichen Abwehrmechanismen vermitteln jedoch in vielen Fällen nur einen unzureichenden Schutzmechanismus gegenüber Pathogenbefall.

Die Ausbildung einer Penetrationsresistenz gegenüber Pathogenen, deren Infektionsmechanismus eine Penetration der Epidermis- oder Mesophyllzellen beinhaltet, ist sowohl für monokotyledone als auch dikotyledone Pflanzen von großer Bedeutung. Sie kann vermutlich im Gegensatz zu anderen Ansätzen zur Resistenzvermittlung die Ausbildung einer Breitspektrum-Resistenz gegen obligat biotrophe, hemibiotrophe und nekrotrophe Pilze ermöglichen.

Bisher wurden zur Erzeugung pilzresistenter Pflanzen häufig quantitative Resistenzmerkmale (Resistenz-QTLs) eingekreuzt. Dieses Verfahren hat aber den Nachteil, dass oft unerwünschte Merkmale mit eingekreuzt werden. Darüber hinaus sind die dazu benötigten Züchtungsmethoden sehr kompliziert und zeitaufwendig.

Daher lag der vorliegenden Erfindung die Aufgabe zugrunde, ein Verfahren zur Erhöhung der Resistenz von Pflanzen gegen penetrierende Pathogene bereitzustellen.

Die Aufgabe wird durch die in den Ansprüchen charakterisierten Ausführungsformen gelöst.

Folglich betrifft die vorliegende Erfindung ein Verfahren zur Erhöhung der Pathogenresistenz in transgenen Pflanzen und/oder Pflanzenzellen, wobei eine DNA-Sequenz, die für ein Protein kodiert, welches eine erhöhte Pilzpathogenresistenz vermittelt, in die Pflanze bzw. Pflanzenzelle eingeführt und dort exprimiert wird.

Im Rahmen einer TIGS (=Transient Induced Gene Silencing)-Analyse in Gerste nach der Methode von Schweizer et al. (2001) wurde gefunden, dass durch ein dsRNAivermitteltes Silencing des Gens RNR8 die Empfänglichkeit der Pflanze für das Pilzpathogen *Blumeria graminis* erhöht ist und daher das Gen RNR8 eine Rolle bei der Vermittlung der Pathogenresistenz von Gerstepflanzen spielen könnte.

RNR8 gehört zu der Familie der leucine-rich-repeat (LRR)-enthaltenden Proteine mit einer Kinasedomäne bzw. einer Kinaseaktivität. Mitglieder dieser Familie spielen in verschiedenen zellulären Prozessen wie der Regulation der Endosperm- und Pollenentwicklung (Li und Wurtzel (1998) Plant Mol. Biol. 37: 749-761; Muschietti et al. (1998) Plant Cell 10: 319-330), der Regulation der Meristem- und Blütenentwicklung (Torii et al. (1996) Plant Cell 8: 735-746; Clark et al. (1997) Cell 89: 575-585; Kim et al. (2000) Plant Sci. 152: 17-26) und dem Gibberellininduzierten Sprosswachstum (van der Knapp et al. (1999) Plant Physiol. 120: 559-569) eine wichtige Rolle. Die LRR-Domäne umfasst 2 bis 45 Wiederholungen einer Aminosäuresequenz mit 20 bis 30 Aminosäuren und faltet sich im Allgemeinen in Hufeisenform.

Von einem weiteren Mitglied dieser Familie, OsXa21 aus Reis, konnte gezeigt werden, dass es Resistenz gegenüber dem bakteriellen Pathogen *Xanthomonas oryzae* vermittelt (Song et al. (1995) Science 270(5243): 1804-1806). Das erfindungsgemäße Protein weist zu OsXa21 nur eine sehr schwache Sequenzhomologie von etwa 23% auf.

Die beschriebenen Proteine vermitteln eine Erhöhung Resistenz gegen Pilzpathogene in Pflanzen. Sie zeichnen sich bevorzugt dadurch aus, dass sie entweder eine leucine-rich-repeat (LRR)-Domäne oder eine Kinaseaktivität aufweisen, besonders bevorzugt weisen die beschriebenen Proteine beide Merkmale auf. Ohne an eine Hypothese gebunden sein zu wollen, wird gegenwärtig davon ausgegangen, dass entweder die Kinaseaktivität oder die LRR-Domäne oder beide Merkmale gemeinsam für die Vermittlung der Pathogenresistenz verantwortlich sind. Im Folgenden wird der Einfachheit halber der Begriff des "beschriebenen Proteins" verwendet.

Die Nukleinsäuresequenz, die im Verfahren der vorliegenden Erfindung in die Pflanze bzw. Pflanzenzelle eingeführt wird und die für ein beschriebenes Protein kodiert, ist ausgewählt aus der Gruppe bestehend aus:
i) Nukleinsäuresequenzen umfassend Nukleotidsequenzen, die den kodierenden Sequenzen von SEQ ID No. 1 entsprechen,
ii) Nukleinsäuresequenzen umfassend Nukleotidsequenzen, die für ein Protein mit der in SEQ ID No. 2 angegebenen Aminosäuresequenz kodieren,
iii) Nukleinsäuresequenzen umfassend Nukleotidsequenzen, die zu den kodierenden Sequenzen von SEQ ID No. 1 eine Sequenzidentität von mindestens 90% über die jeweils gesamte Sequenzlänge aufweisen, und/oder
iv) Nukleinsäuresequenzen umfassend Nukleotidsequenzen, die unter stringenten Bedingungen mit einem komplementären Strang einer Nukleotidsequenz von i) bis iii) hybridisieren.

"Resistenz" bedeutet das Verhindern, das Reprimieren, das Vermindern oder Abschwächen von Krankheitssymptomen einer Pflanze, die infolge eines Befalls durch ein Pathogen auftreten. Die Symptome können vielfältiger Art sein, umfassen aber bevorzugt solche, die direkt oder indirekt zu einer Beeinträchtigung der Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

In einer bevorzugten Ausführungsform werden die folgenden Krankheitssymptome abgeschwächt, vermindert oder verhindert: Bildung von Pusteln und Sporienlagern auf den Oberflächen der befallenen Gewebe, Mazeration der Gewebe, spreitende Nekrosen des Gewebes, Akkumulation von Mykotoxinen z.B. aus *Fusarium graminearum* oder *F*. *culmorum.*

Eine "erhöhte Pilzpathogenresistenz" bedeutet, dass die Abwehrmechanismen einer bestimmten Pflanze oder in einem Teil einer Pflanze, z.B. in einem Organ, einem Gewebe, einer Zelle oder einem Organell, durch Anwendung des erfindungsgemäßen Verfahrens im Vergleich zu einer geeigneten Kontrolle, z.B. dem Wildtyp der Pflanze ("Kontrollpflanze", "Ausgangspflanze"), auf den das erfindungsgemäße Verfahren nicht angewendet wurde, unter ansonsten gleichen Bedingungen (wie beispielsweise Klima- oder Anbaubedingungen, Pathogenart etc.) eine erhöhte Resistenz gegen Mehltau aufweist. Vorzugsweise weisen in einer Pflanze mindestens die Epidermis und/oder Mesophyll-Gewebe oder die Organe, die ein Epidermis und/oder Mesophyll-Gewebe besitzen, eine erhöhte Resistenz gegen Mehltau auf. Beispielsweise ist die Resistenz in den Blättern erhöht.

In einer Ausführungsform wird die Resistenz in Lemma (Deckspelze), palea (Vorspelze), und/oder Glume (Antherenanlage) erhöht.

Die erhöhte Resistenz äußert sich bevorzugt in einer verminderten Ausprägung der Krankheitssymptome, wobei die Krankheitssymptome - neben den oben erwähnten Beeinträchtigungen - auch beispielsweise die Penetrationseffizienz eines Pathogens in die Pflanze oder pflanzliche Zelle oder die Proliferationseffizienz des Pathogens in oder auf denselben umfassen. Dabei sind die Krankheitssymptome bevorzugt um mindestens 10 % oder mindestens 20 %, besonders bevorzugt um mindestens 40 % oder 60 %, insbesondere bevorzugt um mindestens 70 % oder 80 %, am meisten bevorzugt um mindestens 90 % oder 95 % gegenüber der Kontrollpflanze vermindert.

"Pathogen" meint im Rahmen der vorliegenden Anmeldung Organismen, deren Interaktionen mit einer Pflanze zu den oben beschriebenen Krankheitssymptomen führen, insbesondere meint Pathogene Organismen aus dem Reich der Pilze. Vorzugsweise handelt es sich bei dem Pathogen um ein Epidermis- bzw. Mesophyllzellen penetrierendes Pathogen, besonders bevorzugt um Pathogene, die über Spaltöffnungen in Pflanzen eindringen und anschließend Mesophyllzellen penetrieren. Bevorzugt sind dabei Organismen der Stämme Ascomycota und Basidomycota genannt. Besonders bevorzugt sind dabei die Familien Blumeriaceae, Pucciniaceae, Mycosphaerellaceae und Hypocreaceae.

Besonders bevorzugt sind Organismen dieser Familien, die zu den Gattungen Blumeria, Puccinia, Fusarium oder Mycosphaerella zählen.

Ganz besonders bevorzugt sind die Arten *Blumeria graminis, Puccinia triticina, Puccinia striiformis, Mycosphaerella graminicola, Stagonospora nodorum, Fusarium graminearum, Fusarium culmorum, Fusarium avenaceum, Fusarium poae* und *Microdochium nivale.*

Ebenfalls beschrieben ist ein isoliertes Nukleinsäuremolekül, enthaltend eine Nukleinsäuresequenz ausgewählt aus der Gruppe bestehend aus:
i) Nukleinsäuresequenzen umfassend Nukleotidsequenzen, die den kodierenden Sequenzen von SEQ ID No. 1 oder Fragmenten davon entsprechen,
ii) Nukleinsäuresequenzen umfassend Nukleotidsequenzen, die für ein Protein mit der in SEQ ID No. 2 angegebenen Aminosäuresequenz oder Fragmenten davon kodieren,
iii) Nukleinsäuresequenzen umfassend Nukleotidsequenzen, die zu den kodierenden Sequenzen von SEQ ID No. 1 eine Sequenzidentität von mindestens 60% aufweisen, und/oder
iv) Nukleinsäuresequenzen umfassend Nukleotidsequenzen, die unter stringenten Bedingungen mit einem komplementären Strang einer Nukleotidsequenz von i) bis iii) hybridisieren,
die für ein Protein kodiert, welches in Pflanzen eine erhöhte Pathogenresistenz, bevorzugt eine erhöhte Pilzpathogenresistenz vermittelt.

Der Begriff "Nukleinsäure(molekül)", wie hier verwendet, umfasst in einer bevorzugten Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Molekül z.B. weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren. Bei allen hier erwähnten Nukleinsäuremolekülen kann es sich z.B. um RNA, DNA oder cDNA handeln.

Die im Verfahren verwendeten Nukleinsäuremoleküle, z.B. ein Nukleinsäuremolekül mit einer Nukleotidsequenz der SEQ ID No. 1 oder einem Teil davon, können unter Verwendung molekularbiologischer Standardtechniken und der hier bereit gestellten Sequenzinformation isoliert werden. Auch können mit Hilfe von Vergleichsalgorithmen, wie sie z.B. auf der NCBI-Homepage unter http://www.ncbi.nlm.nih.gov zu finden sind, beispielsweise eine homologe Sequenz oder homologe, konservierte Sequenzbereiche auf DNA- oder Aminosäureebene identifiziert werden. Wesentliche Teile dieser Sequenz oder die gesamte homologe Sequenz können als Hybridisierungssonde unter Verwendung von Standardhybridisierungstechniken (wie z.B. beschrieben in Sambrook et al., vide supra) zur Isolierung weiterer im Verfahren nützlicher Nukleinsäuresequenzen aus anderen Organismen durch das Screening von cDNA- und/oder genomischen Banken verwendet werden. Überdies lässt sich ein Nukleinsäuremolekül, umfassend eine vollständige Sequenz gemäß SEQ ID No. 1 oder einen Teil davon, durch Polymerasekettenreaktion isolieren, wobei Oligonukleotidprimer auf der Basis der hier angegebenen Sequenzen oder von Teilen davon verwendet werden (z.B. kann ein Nukleinsäuremolekül, umfassend die vollständige Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18: 5294 - 5299) und daraus mittels reverser Transkriptase (z.B. Moloney-MLV-Reverse Transkriptase, erhältlich von Gibco/BRL, Bethesda, MD oder AMV-Reverse-Transkriptase, erhältlich von Seikagaku Amerika, Inc., St. Petersburg, FL) cDNA herstellen. Synthetische Oligonukleotidprimer zur Amplifizierung mittels Polymerasekettenreaktion lassen sich auf der Basis der in SEQ ID No. 1 dargestellten Nukleinsäuresequenz oder mit Hilfe der in SEQ ID No. 2 dargestellten Aminosäuresequenz erstellen. Eine beschriebene Nukleinsäure kann unter Verwendung von cDNA oder alternativ von genomischer DNA als Matrize und geeigneten Oligonukleotidprimem mittels Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Nukleotidsequenz entsprechen, welche für ein beschriebenes Protein kodiert, können durch Standardsyntheseverfahren, beispielsweise mit einem automatischen DNA-Synthesegerät, hergestellt werden.

Unter dem Begriff "Sequenzidentität" zwischen zwei Nukleinsäuresequenzen wird die Identität der Nukleinsäuresequenz über die jeweils gesamte Sequenzlänge verstanden, in einer bevorzugten Ausführungsform über die gesamte exprimierte Sequenzlänge, bevorzugt cDNA, noch mehr bevorzugt über die codierende Sequenz, bevorzugt CDS, verstanden, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25: 3389ff) unter Einstellung folgender Parameter berechnet wird:

| | |
|---|---|
| Gap Weight: 50 | Length Weight: 3 |
| Average Match: 10 | Average Mismatch: 0 |

Beispielhaft weist eine Sequenz, die eine Homologie von mindestens 80 % auf Nukleinsäurebasis mit der Sequenz SEQ ID NO: 1 aufweist, bei einem Vergleich mit der Sequenz nach SEQ ID No. 1 nach obigem Programmalgorithmus mit obigem Parametersatz eine Homologie von mindestens 80 % auf.

Verwendet werden Nukleinsäuresequenzen, die zu der in SEQ ID No. 1 angegebenen Sequenz eine Sequenzidentität von mindestens 90% und bevorzugt von mindestens 92, 94, 96, 98 oder 99% aufweisen.

Unter "Identität zwischen zwei Proteinen" wird die Identität der Aminosäuren über einen bestimmten Proteinbereich verstanden, vorzugsweise die gesamte Proteinlänge, insbesondere die Identität, die durch Vergleich mit Hilfe von Software, z.B. der Lasergene-Software der Firma DNA Star Inc., Madison, Wisconsin (USA) unter Anwendung der CLUSTAL-Methode (Higgins et al. (1989) Comput. Appl. Biosci. 5(2): 151) berechnet wird. Homologien können ebenfalls mit Hilfe der Lasergene-Software der Firma DNA Star Inc., Madison, Wisconsin (USA) unter Anwendung der CLUSTAL-Methode (Higgins et al. (1989) Comput. Appl. Biosci. 5(2): 151) berechnet werden.

Bevorzugt wird unter "Identität zwischen zwei Proteinen" die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

"Standardhybridisierungsbedingungen" ist breit zu verstehen und meint je nach Anwendung stringente oder weniger stringente Hybridisierungsbedingungen. Solche Hybridisierungsbedingungen sind unter anderem bei Sambrook und Russell, Molecular Cloning - A Laboratory Manual, 3. Auflage, Cold Spring Harbor Laboratory Press, 2001) oder in Current Protocols in Molecular Biology, John Wiley &Sons, N.Y. (1989), 6.3.1-6.3.6. beschrieben.

Der Fachmann würde Hybridisierungsbedingungen auswählen, die es ihm ermöglichen, spezifische von unspezifischen Hybridisierungen zu unterscheiden.

Beispielhaft können die Bedingungen während des Waschschrittes ausgewählt sein aus Bedingungen mit geringer Stringenz (mit ungefähr 2X SSC bei 50°C) und solchen mit hoher Stringenz (mit ungefähr 0.2X SSC bei 50°C, bevorzugt bei 65°C) (20X SSC: 0,3M Natriumcitrat, 3M NaCl, pH 7.0). Darüber hinaus kann die Temperatur während des Waschschrittes von niedrig stringenten Bedingungen bei Raumtemperatur, d.h. ungefähr 22°C, bis zu stärker stringenten Bedingungen bei ungefähr 65°C angehoben werden. Beide Parameter, Salzkonzentration und Temperatur, können gleichzeitig oder auch einzeln variiert werden, wobei der jeweils andere Parameter konstant gehalten wird. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt. Einige beispielhafte Bedingungen für Hybridisierung und Waschschritt sind infolge gegeben:
(1) Hybridisierungbedingungen können zum Beispiel aus nachfolgenden Bedingungen ausgewählt sein:
   a) 4X SSC bei 65°C,
   b) 6X SSC bei 45°C,
   c) 6X SSC, 100 µg/ml denaturierte, fragmentierte Fischsperma-DNA bei 68°C,
   d) 6X SSC, 0,5 % SDS, 100 µg/ml denaturierte, fragmentierte Lachssperma-DNA bei 68°C,
   e) 6X SSC, 0,5 % SDS, 100 µg/ml denaturierte, fragmentierte Lachssperma-DNA, 50 % Formamid bei 42°C.
   f) 50 % Formamid, 4X SSC bei 42°C,
   g) 50 % (vol/vol) Formamid, 0,1% Rinderserumalbumin, 0,1 % Ficoll, 0,1 % Polyvinylpyrrolidon, 50 mM Natriumphosphatpuffer pH 6,5, 750 mM NaCl, 75 mM Natriumcitrat bei 42°C,
   h) 2X oder 4X SSC bei 50°C (schwach stringente Bedingung),
   i) 30 bis 40 % Formamid, 2X oder 4X SSC bei 42°C (schwach stringente Bedingung).
   j) 500 mM Natriumphosphatpuffer pH 7,2, 7% SDS (g/V), 1mM EDTA, 10µg/ml single stranded DNA, 0,5% BSA (g/V) (Church und Gilbert (1984) Proc. Natl. Acad.Sci. U.S.A. 81(7): 1991-1995.)
(2) Waschschritte können zum Beispiel aus nachfolgenden Bedingungen ausgewählt sein:
   a) 0,015 M NaCl/0,0015 M Natriumcitrat/0,1 % SDS bei 50°C,
   b) 0.1X SSC bei 65°C,
   c) 0,1X SSC, 0,5 % SDS bei 68°C,
   d) 0,1X SSC, 0,5 % SDS, 50 % Formamid bei 42°C,
   e) 0,2X SSC, 0,1 % SDS bei 42°C,
   f) 2X SSC bei 65°C (schwach stringente Bedingung).

In einer Ausführungsform werden die Hybridisierungsbedingungen wie folgt gewählt:
Es wird ein Hybridisierungspuffer gewählt, der Formamid, NaCl und PEG 6000 enthält. Die Anwesenheit von Formamid im Hybridisierungspuffer destabilisiert doppelsträngige Nukleinsäuremoleküle, wodurch die Hybridisierungstemperatur auf 42°C gesenkt werden kann, ohne dadurch die Stringenz zu erniedrigen. Die Verwendung von Salz im Hybridisierungspuffer erhöht die Renaturierungsrate einer Duplex, bzw. die Hybridisierungseffizienz. Obwohl PEG die Viskosität der Lösung erhöht, was einen negativen Einfluss auf Renaturierungsraten ausübt, wird durch die Anwesenheit des Polymers in der Lösung die Konzentration der Sonde im verbleibenden Medium erhöht, was die Hybridisierungsrate steigert. Die Zusammensetzung des Puffers ist wie folgt:

| Hybridisierungspuffer |
|---|
| 250 mM Natriumphosphat-Puffer pH 7,2 |
| 1 mM EDTA |
| 7 % SDS (g/v) |
| 250 mM NaCl |
| 10 µg/ml ssDNA |
| 5 % Polyethylenglykol (PEG) 6000 |
| 40 % Formamid |

Die Hybridisierungen werden bei 42°C über Nacht durchgeführt. Die Filter werden am nächsten Morgen 3x mit 2xSSC + 0,1 % SDS für jeweils ca. 10 min. gewaschen.

Nukleinsäuresequenzen, die von der in SEQ ID No.1 angegebenen Nukleinsäuresequenz abweichen, können z.B. durch Einbringen einer oder mehrerer Nukleotidsubstitutionen, -additionen oder -deletionen in eine Nukleotidsequenz der SEQ ID No. 1 erzeugt werden, so dass Proteine entstehen, in die gegenüber der in SEQ ID No. 2 angegebenen Sequenz eine oder mehrere Aminosäuresubstitutionen, -additionen oder -deletionen eingebracht wurden. Mutationen können in die Sequenz der SEQ ID No. 1 durch Standardtechniken, wie z.B. stellenspezifische Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste, also an Aminosäureresten, die die Kinaseaktivität und/oder die LRR-Domäne nicht beeinflussen, hergestellt. Bei einer "konservativen Aminosäuresubstitution" wird ein Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure und Glutaminsäure), ungeladenen polaren Seitenketten (z.B. Glycin, Asparagin, Glutamin, Serin, Threonin, Tyrosin, Cystein), unpolaren Seitenketten (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan). Ein vorhergesagter nicht-essentieller Aminosäurerest in dem erfindungsgemäß verwendeten Protein wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der für das erfindungsgemäße Protein kodierenden Sequenz eingebracht werden, z.B. hinsichtlich ihrer Fähigkeit, Pathogenresistenz zu vermitteln, durchmustert werden.

Unter im Wesentlichen gleicher enzymatischer Aktivität des im erfindungsgemäßen Verfahren verwendeten Proteins mit Kinaseaktivität ist zu verstehen, dass die enzymatische Aktivität gegenüber den durch die Sequenz mit SEQ ID No. 1 oder deren Derivate kodierten Enzymen im Vergleich noch mindestens 50%, bevorzugt mindestens 60%, besonders bevorzugt mindestens 70%, insbesondere bevorzugt mindestens 80% und am meisten bevorzugt mindestens 90% beträgt. Proteine mit Kinaseaktivität mit im Wesentlichen gleicher enzymatischer Aktivität sind damit ebenfalls geeignet, in transgenen Pflanzen eine erhöhte Pathogenresistenz zu bewirken.

Die Kinase-Aktivität von Proteinen lässt sich durch einfache Verfahren, die dem Fachmann bekannt sind und die als Kinase-Assays bezeichnet werden, bestimmen. Dazu wird z.B. das zu testende Protein, das z.B. durch Immunpräzipitation aufgereinigt wurde, in einem geeigneten Puffer mit radioaktiv markiertem ATP als Phosphatdonor und einem geeigneten Substrat inkubiert und anschließend das Substrat über ein SDS-Polyacrylamid-Gel aufgetrennt. Wenn das Protein eine Kinase-Aktivität aufweist, wird das Substrat mit dem radioaktiv markiertes Phosphat markiert und die Radioaktivität kann mit geeigneten Verfahren nachgewiesen und quantifiziert werden.

Eine Erhöhung der Pathogenresistenz kann auch dadurch erreicht werden, dass die Expression des Pflanzen-eigenen endogenen Proteins, welches dem beschriebenen Protein entspricht, manipuliert wird. Es handelt sich also um ein Pflanzen-eigenes Protein, das eine Erhöhung der Pathogenresistenz vermittelt und das bevorzugt eine Kinase-Aktivität oder eine LRR-Domäne und besonders bevorzugt beide Merkmale aufweist. Diese Manipulation der Proteinexpression kann beispielsweise durch eine Veränderung der Promotor-DNA-Sequenz des Protein-kodierenden Gens erreicht werden. Eine solche Veränderung, die eine veränderte, vorzugsweise erhöhte Expressionsrate des endogenen beschriebenen Gens zur Folge hat, kann durch Deletion oder Insertion von DNA-Sequenzen erfolgen. Eine Veränderung der Promotor-Sequenz von endogenen beschriebenen Genen führt in der Regel zu einer Veränderung der exprimierten Menge des Gens und damit z.B. auch zu einer Veränderung der in der Zelle bzw. den Pflanzen nachweisbaren Kinase-Aktivität (sofern das Protein eine Kinaseaktivität aufweist). Die Veränderung der Promotor-Sequenz des beschriebenen endogenen Gens kann auch zu einer Veränderung der Menge an Protein mit einer LRR-Domäne in der Zelle führen.

Eine weitere Möglichkeit zur Erhöhung der Aktivität und des Gehalts des beschriebenen endogenen Proteins besteht darin, Transkriptionsfaktoren, die in die Transkription des entsprechenden endogenen Genes involviert sind, z.B. durch Überexpression hochzuregulieren. Die Maßnahmen zur Überexpression von Transkriptionsfaktoren sind dem Fachmann bekannt.

Des Weiteren kann eine erhöhte Expression eines endogenen erfindungsgemäßen Gens dadurch erzielt werden, dass ein im nicht-transformierten Organismus nicht vorkommendes Regulatorprotein mit dem Promotor dieser Gene in Wechselwirkung tritt. Bei einem solchen Regulator kann es sich um ein chimäres Protein handeln, welches aus einer DNA-Bindedomäne und einer Transkriptionsaktivator-Domäne besteht, wie beispielsweise in WO 96/06166 beschrieben.

"Transgen" meint zum Beispiel bezüglich einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor, alle solche durch gentechnische Methoden zustande gekommenen Konstruktionen oder Organismen, in denen sich entweder
a) die RNR8-Nukleinsäuresequenz, oder
b) eine mit der RNR8-Nukleinsäuresequenz funktionell verknüpfte genetische Kontrollsequenz, zum Beispiel ein Promotor, oder
c) (a) und (b)
nicht in ihrer natürlichen, genetischen Umgebung befinden oder durch gentechnische Methoden modifiziert wurden, wobei die Modifikation beispielhaft eine Substitution, Addition, Deletion, oder Insertion eines oder mehrerer Nukleotidreste sein kann. "Natürliche genetische Umgebung" meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des RNR8-Promotors mit dem entsprechenden RNR8-Gen - wird zu einer transgenen Expressionskassette, wenn diese durch nichtnatürliche, synthetische ("künstliche") Verfahren wie beispielsweise eine Mutagenisierung verändert wird. Entsprechende Verfahren sind beschrieben (US 5,565,350; WO 00/15815).

"Einführung" umfasst im Rahmen der Erfindung alle Verfahren, die dazu geeignet sind, eine RNR8-Nukleinsäuresequenz, direkt oder indirekt, in eine Pflanze oder eine Zelle, Kompartiment, Gewebe, Organ oder Samen derselben einzuführen oder dort zu generieren. Die Einführung kann zu einer vorübergehenden (transienten) oder zu einer dauerhaften (stabilen) Präsenz einer RNR8-Nukleinsäuresequenz führen.

"Einführung" umfasst beispielsweise Verfahren wie Transfektion, Transduktion oder Transformation.

Die Einbringung einer erfindungsgemäßen Expressionskassette in einen Organismus oder Zellen, Gewebe, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die Expressionskassetten enthalten sind. Die Expressionskassette kann in den Vektor (zum Beispiel ein Plasmid) über eine geeignete Restriktionsschnittstelle eingeführt werden. Das entstandene Plasmid wird zunächst in *E*.*coli*-Zellen eingeführt. Korrekt transformierte *E*.*coli*-Zellen werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Bei den Vektoren kann es sich beispielsweise um Plasmide, Cosmide, Phagen, Viren oder auch Agrobakterien handeln. In einer vorteilhaften Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Die Herstellung eines transformierten Organismus (bzw. einer transformierten Zelle) erfordert, dass das entsprechende DNA-Molekül in die entsprechende Wirtszelle eingebracht wird und anschließend durch die Genexpression die entsprechenden RNAs und Proteine gebildet werden.

Für diesen Vorgang, der als Transformation (oder Transduktion bzw. Transfektion) bezeichnet wird, steht eine Vielzahl von Methoden zur Verfügung (Keown et al. (1990) Methods in Enzymology 185: 527-537). So kann die DNA oder RNA beispielhaft direkt durch Mikroinjektion oder durch Bombardierung mit DNAbeschichteten Mikropartikeln eingeführt werden. Auch kann die Zelle chemisch, zum Beispiel mit Polyethylenglykol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen in die Zelle eingeführt werden. Elektroporation ist eine weitere geeignete Methode zur Einführung von DNA, bei der die Zellen reversibel durch einen elektrischen Impuls permeabilisert werden. Entsprechende Verfahren sind beschrieben (beispielsweise bei Bilang et al. (1991) Gene 100: 247-250; Scheid et al. (1991) Mol. Gen. Genet. 228: 104-112; Guerche et al. (1987) Plant Science 52: 111-116; Neuhause et al. (1987) Theor. Appl. Genet. 75: 30-36; Klein et al. (1987) Nature 327: 70-73; Howell et al. (1980) Science 208: 1265; Horsch et al.(1985) Science 227: 1229-1231; DeBlock et al. (1989) Plant Physiology 91: 694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardment" Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels *Agrobacterium tumefaciens* oder *Agrobacterium rhizogenes* durchgeführt werden. Die Verfahren sind beispielsweise beschrieben bei Horsch et al. (1985) Science 225: 1229f.

Werden Agrobakterien verwendet, so ist die Expressionskassette in spezielle Plasmide zu integrieren, bei denen es sich entweder um einen Zwischenvektor (englisch: shuttle or intermediate vector) oder um einen binären Vektor handeln kann. Wird ein Ti- oder Ri-Plasmid zur Transformation verwendet, ist zumindest die rechte Begrenzung, meistens jedoch sowohl die rechte als auch die linke Begrenzung der Ti- oder Ri-Plasmid-T-DNA als flankierende Region mit der einzuführenden Expressionskassette verbunden.

Bevorzugt werden binäre Vektoren verwendet. Binäre Vektoren können sowohl in *E.coli* als auch in Agrobacterium replizieren. Sie enthalten in der Regel ein Selektionsmarkergen und einen Linker oder Polylinker flankiert von der rechten und linken T-DNA-Begrenzungssequenz. Sie können direkt in Agrobacterium transformiert werden (Holsters et al. (1978) Mol. Gen. Genet. 163: 181-187). Das Selektionsmarkergen, zum Beispiel das nptII Gen, das eine Resistenz gegen Kanamycin verleiht, erlaubt eine Selektion transformierter Agrobakterien. Das in diesem Fall als Wirtsorganismus fungierende Agrobacterium sollte bereits ein Helfer-Ti-Plasmid mit der vir-Region enthalten, die für die Übertragung der T-DNA auf die pflanzliche Zelle erforderlich ist. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4: 277-287). Verschiedene binäre Vektoren sind bekannt und teilweise kommerziell erhältlich wie zum Beispiel pBI101.2 oder pBIN19 (Clontech Laboratories, Inc. USA).

Im Falle der Injektion oder Elektroporation von DNA bzw. RNA in pflanzliche Zellen werden keine besonderen Anforderungen an das verwendete Plasmid gestellt. Einfache Plasmide wie die der pUC-Reihe können verwendet werden. Sollen aus den transformierten Zellen vollständige Pflanzen regeneriert werden, so ist es erforderlich, dass sich auf dem Plasmid ein zusätzliches selektionierbares Markergen befindet.

Stabil transformierte Zellen, d.h. solche, die die eingeführte DNA integriert in die DNA der Wirtszelle enthalten, können von untransformierten unterschieden werden, wenn ein selektionierbarer Marker Bestandteil der eingeführten DNA ist (McCormick et al. (1986) Plant Cell Reports 5: 81-84). Als Marker kann beispielhaft jedes Gen fungieren, das eine Resistenz gegen Antibiotika oder Herbizide (wie Kanamycin, G 418, Bleomycin, Hygromycin oder Phosphinotricin) zu verleihen vermag. Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiele umfassen das bar-Gen, das Resistenz gegen das Herbizid Phosphinotricin verleiht (Rathore et al. (1993) Plant Mol Biol 21(5): 871-884), das nptII-Gen, das Resistenz gegen Kanamycin verleiht, das hpt-Gen, das Resistenz gegen Hygromycin verleiht, oder das EPSP-Gen, das Resistenz gegen das Herbizid Glyphosat verleiht. Die erhaltenen Pflanzen können in üblicher Weise gezüchtet und gekreuzt werden. Es sollten zwei oder mehr Generationen kultiviert werden, um sicherzustellen, dass die genomische Integration stabil und vererblich ist.

Die oben genannten Verfahren sind beispielsweise beschrieben in Jenes et al. (1993) Techniques for Gene Transfer, in: Transgenic Plants, Vol. 1, Engineering and Utilization, herausgegeben von SD Kung und R Wu, Academic Press, S. 128-143 sowie in Potrykus (1991) Annu. Rev. Plant Physiol. Plant Molec. Biol. 42: 205-225). Vorzugsweise wird das zu exprimierende Konstrukt in einen Vektor kloniert, der geeignet ist, *Agrobacterium tumefaciens* zu transformieren, beispielsweise in pBin19 (Bevan et al. (1984) Nucl. Acids Res. 12: 8711f).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Dem Fachmann sind auch Verfahren bekannt, um aus Pflanzenzellen Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14: 273-278; Jahne et al. (1994) Theor. Appl. Genet. 89: 525-533 verwendet.

Ein weiterer Gegenstand der vorliegenden Erfindung ist ein rekombinantes Nukleinsäuremolekül, umfassend die folgenden Elemente in 5'-3'-Orientierung:
- regulatorische Sequenzen eines in Pflanzenzellen aktiven Promotors,
- operativ daran gebunden eine beschriebene DNA-Sequenz,
- ggf. operativ daran gebunden regulatorische Sequenzen, die in der Pflanzenzelle als Transkriptions-, Terminations- und/oder Polyadenylierungssignale dienen können.

"Operativ daran gebunden" bedeutet, dass ein Promotor und die zu exprimierende Nukleinsäuresequenz und ggf. weitere regulatorische Elemente derart angeordnet sind, dass jedes der regulatorischen Elemente seine Funktion bei der Expression der Nukleinsäuresequenz ausüben kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter der als Promotor fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare und am meisten bevorzugt kleiner als 50 Basenpaare.

Die Herstellung einer operativen Verknüpfung und die Herstellung eines rekombinanten Nukleinsäuremoleküls kann mittels gängiger Rekombinations- und Klonierungstechniken erfolgen, wie sie beispielsweise in Sambrook und Russell (2001) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen Promotor und der zu exprimierenden Nukleinsäuresequenz können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann das rekombinanten Nukleinsäuremolekül, mindestens umfassend einen Promotor und die zu exprimierende Nukleinsäuresequenz in operativer Verknüpfung, integriert in einem Vektor vorliegen und durch zum Beispiel Transformation in ein pflanzliches Genom insertiert werden.

Pflanzenspezifische Promotoren meint grundsätzlich jeden Promotor, der die Expression von Genen, insbesondere Fremdgenen, in Pflanzen oder Pflanzenteilen, - zellen, -geweben, -kulturen steuern kann. Dabei kann die Expression beispielsweise konstitutiv, induzierbar oder entwicklungsabhängig sein.

Bevorzugt sind:

### a) Konstitutive Promotoren

"Konstitutiver" Promotor meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten. Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV (Blumenkohlmosaikvirus) (Franck et al. (1980) Cell 21: 285-294; Odell et al. (1985) Nature 313: 810-812; Shewmaker et al. (1985) Virology 140: 281-288; Gardner et al. (1986) Plant Mol. Biol. 6: 221- 228) oder der 19S CaMV-Promotor (US 5,352,605; WO 84/02913; Benfey et al. (1989) EMBO J. 8: 2195-2202). Ein weiterer geeigneter konstitutiver Promotor ist der "Rubisco small subunit (SSU)"-Promotor (US 4,962,028), der Promotor der Nopalinsynthase aus Agrobacterium, der TR-Doppelpromotor, der OCS (Octopin Synthase)-Promotor aus Agrobacterium, der Ubiquitin-Promotor (Holtorf S et al. (1995) Plant Mol Biol 29: 637-649), der Ubiquitin 1-Promotor (Christensen et al. (1992) Plant Mol Biol 18: 675-689; Bruce et al. (1989) Proc. Natl. Acad. Sci. USA 86: 9692-9696), der Smas-Promotor, der Cinnamylalkoholdehydrogenase-Promotor (US 5,683,439), die Promotoren der vakuolärer ATPase Untereinheiten oder der Promotor eines prolinreichen Proteins aus Weizen (WO 91/13991), sowie weitere Promotoren von Genen, deren konstitutive Expression in Pflanzen dem Fachmann bekannt ist. Als konstitutiver Promotor insbesondere bevorzugt ist der Promotor des Nitrilase-1 (nit1) Gens aus *A. thaliana* (GenBank Acc.-No.: Y07648.2, Nukleotide 2456-4340, Hillebrand et al. (1996) Gene 170:197-200).

### b) Gewebespezifische Promotoren

In einer Ausführungsform werden Promotoren mit Spezifitäten für die Antheren, Ovarien, Blüten, Blätter, Stengel, Wurzeln und Samen verwendet. Samenspezifische Promotoren sind zum Beispiel der Promotor des Phaseolins (US 5,504,200; Bustos et al. (1989) Plant Cell 1(9): 839-53), des 2S-Albumingens (Joseffson et al. (1987) J. Biol. Chem. 262: 12196-12201), des Legumins (Shirsat et al. (1989) Mol. Gen. Genet. 215(2): 326-331), des USP (unknown seed protein; Bäumlein et al. (1991) Mol. Gen. Genet. 225(3): 459-67), des Napin-Gens (US 5,608,152; Stalberg et al. (1996) L. Planta 199: 515-519), des Gens kodierend für das Saccharosebindeprotein (WO 00/26388) oder der Legumin B4-Promotor (LeB4; Bäumlein et al. (1991) Mol. Gen. Genet. 225: 121-128; Bäumlein et al. (1992) Plant Journal 2(2): 233-9; Fiedler et al. (1995) Biotechnology (NY) 13(10): 1090f), der Oleosin-Promotor aus Arabidopsis (WO 98/45461), der Bce4-Promotor aus Brassica (WO 91/13980). Weitere geeignete samenspezifische Promotoren sind die der Gene kodierend für das "High Molecular Weight Glutenin" (HMWG), Gliadin, Verzweigungsenzym, ADP-Glucose-Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promotor des lpt2- oder des lpt1-Gens (WO 95/15389, WO 95/23230) oder die in WO 99/16890 beschriebenen Promotoren (Promotoren des Hordein-Gens, des Glutelin-Gens, des Oryzin-Gens, des Prolamin-Gens, des Gliadin-Gens, des Zein-Gens, des Kasirin-Gens oder des Secalin-Gens).

Knollen-, Speicherwurzel- oder Wurzel-spezifische Promotoren sind beispielsweise der Patatin-Promotor Klasse I (B33) und der Promotor des Cathepsin D-Inhibitors aus Kartoffel.

Blattspezifische Promotoren sind beispielsweise der Promotor der cytosolischen FBPase aus Kartoffel (WO 97/05900), der SSU Promotor (small subunit) der Rubisco (Ribulose-1,5-bisphosphatcarboxylase) oder der ST-LSI Promotor aus Kartoffel (Stockhaus et al. (1989) EMBO J. 8: 2445-2451). Epidermis-spezifische Promotoren sind beispielsweise der Promotor des OXLP-Gens ("Oxalat-Oxidase like protein"; Wei et al. (1998) Plant Mol. Biol. 36: 101-112), ein Promotor bestehend aus dem GSTA1-Promotor und dem WIR1a-Intron (WO 2005/035766) und der GLP4-Promotor (PCT/EP 2006/062747).

Andere gewebespezifische Promotoren sind z.B. blütenspezifische Promotoren wie beispielsweise der Phytoen-Synthase-Promotor (WO 92/16635) oder der Promotor des Prr-Gens (WO 98/22593) und Antheren-spezifische Promotoren wie der 5126-Promotor (US 5,689,049, US 5,689,051), der globl-Promotor und der γ-Zein Promotor.

### c) Chemisch induzierbare Promotoren

Die Expressionskassetten können auch einen chemisch induzierbaren Promotor enthalten (Übersichtsartikel: Gatz et al. (1997) Annu. Rev. Plant Physiol. Plant Mol. Biol. 48: 89-108), durch den die Expression des exogenen Gens in der Pflanze zu einem bestimmten Zeitpunkt gesteuert werden kann. Derartige Promotoren, wie z.B. der PRP1 Promotor (Ward et al. (1993) Plant Mol. Biol. 22: 361-366), ein durch Salicylsäure induzierbarer Promotor (WO 95/19443), ein durch Benzolsulfonamid induzierbarer Promotor (EP 0 388 186), ein durch Tetrazyklin induzierbarer Promotor (Gatz et al. (1992) Plant J. 2: 397-404), ein durch Abscisinsäure induzierbarer Promotor (EP 0 335 528) bzw. ein durch Ethanol oder Cyclohexanon induzierbarer Promotor (WO 93/21334) können ebenfalls verwendet werden.

### d) Stress- oder Pathogen-induzierbare Promotoren

Ganz besonders vorteilhaft ist die Verwendung von pathogen-induzierbaren Promotoren, da diese eine Expression nur im Bedarfsfall (d.h. Pathogenbefall) ermöglichen.

Im erfindungsgemäßen Verfahren werden daher in einer Ausführungsform in Pflanzen aktive Promotoren verwendet, bei denen es sich um Pathogen-induzierbare Promotor handelt.

Pathogen-induzierbare Promotoren umfassen die Promotoren von Genen, die infolge eines Pathogenbefalls induziert werden, wie beispielsweise Gene von PR-Proteinen, SAR-Proteinen, β-1,3-Glucanase, Chitinase usw. (beispielsweise Redolfi et al. (1983) Neth. J. Plant Pathol. 89: 245-254; Uknes et al. (1992) Plant Cell 4: 645-656; Van Loon (1985) Plant Mol. Virol. 4: 111-116; Marineau et al. (1987) Plant Mol. Biol. 9: 335-342; Matton et al. (1987) Molecular Plant-Microbe Interactions 2: 325-342; Somssich et al. (1986) Proc. Natl. Acad. Sci. USA 83: 2427-2430; Somssich et al. (1988) Mol. Gen. Genetics 2: 93-98; Chen et al. (1996) Plant J. 10: 955-966; Zhang and Sing (1994) Proc. Natl. Acad. Sci. USA 91: 2507-2511; Warner et al. (1993) Plant J. 3: 191-201; Siebertz et al. (1989) Plant Cell 1: 961-968).

Umfasst sind auch durch Verwundung induzierbare Promotoren wie der des pinII-Gens (Ryan (1990) Ann. Rev. Phytopath. 28: 425-449; Duan et al. (1996) Nat. Biotech. 14: 494-498), des wun1- und wun2-Gens (US 5,428,148), des win1- und win2-Gens (Stanford et al. (1989) Mol. Gen. Genet. 215: 200-208), des Systemin-Gens (McGurl et al. (1992) Science 225: 1570-1573), des WIP1-Gens (Rohmeier et al. (1993) Plant Mol. Biol. 22: 783-792; Eckelkamp et al. (1993) FEBS Letters 323: 73-76), des MPI-Gens (Corderok et al. (1994) Plant J. 6(2): 141-150) und dergleichen.

Eine Quelle für weitere pathogen-induzierbare Promotoren stellt die PR-Genfamilie dar. Eine Reihe von Elementen in diesen Promotoren haben sich als vorteilhaft erwiesen. So vermittelt der Nukleotidbereich von Nukleotid -364 bis Nukleotid -288 im Promotor von PR-2d Salicylat-Spezifität (Buchel et al. (1996) Plant Mol. Biol. 30: 493-504). Die Sequenz 5'-TCATCTTCTT-3' taucht im Promotor der Gersten β-1, 3-Glucanase und in mehr als 30 weiteren stress-induzierten Genen wiederholt auf. Diese Region bindet in Tabak ein nukleäres Protein, dessen Menge durch Salicylat erhöht wird. Die PR-1-Promotoren aus Tabak und Arabidopsis (EP-A 0 332 104, WO 98/03536) eignen sich ebenfalls als pathogen-induzierbare Promotoren. Bevorzugt, da besonders spezifisch durch Pathogen induziert, sind die "acidic *PR-5*"-(aPR5)-Promotoren aus Gerste (Schweizer et al. (1997) Plant Physiol. 114: 79-88) und Weizen (Rebmann et al. (1991) Plant Mol. Biol. 16: 329-331). aPR5-Proteine akkumulieren in ca. 4 bis 6 Stunden nach Pathogenbefall und zeigen nur eine sehr geringe Hintergrundexpression (WO 99/66057). Ein Ansatz, um eine erhöhte pathogen-induzierte Spezifität zu erreichen, bildet die Herstellung synthetischer Promotoren aus Kombinationen von bekannten pathogen-responsiven Elementen (Rushton et al. (2002) Plant Cell 14: 749-762; WO 00/01830; WO 99/66057). Weitere pathogen-induzierbare Promotoren aus verschiedenen Arten sind dem Fachmann bekannt (EP-A 1 165 794; EP-A 1 062 356; EP-A 1 041 148; EP-A 1 032 684).

Weitere pathogen-induzierbare Promotoren umfassen den Flachs *Fis1*-Promotor (WO 96/34949), den Vstl-Promotor (Schubert et al. (1997) Plant Mol. Biol. 34: 417-426) sowie den EAS4 Sesquiterpen-Cyclase-Promotor aus Tabak (US 6,100,451).

Ferner sind Promotoren bevorzugt, die durch biotischen oder abiotischen Stress induziert werden, wie beispielsweise der pathogen-induzierbare Promotor des PRP1-Gens (bzw. *gst1* Promotor) z.B. aus Kartoffel (WO 96/28561; Ward et al. (1993) Plant Mol. Biol. 22: 361-366), der Hitze-induzierbare hsp70- oder hsp80-Promotor aus Tomate (US 5,187,267), der Kälte-induzierbare alpha-Amylase-Promotor aus der Kartoffel (WO 96/12814), der Licht-induzierbare PPDK-Promotor oder der verwundungsinduzierte pinII-Promotor (EP-A 0 375 091).

### e) Mesophyllgewebe-spezifische Promotoren

"Mesophyllgewebe" meint das zwischen den Epidermisschichten liegende Blattgewebe, bestehend aus dem Palisadengewebe, dem Schwammgewebe und den Blattadern.

Im erfindungsgemäßen Verfahren werden in einer Ausführungsform Mesophyllgewebe-spezifische Promotoren wie beispielsweise der Promotor des Weizen Germin 9f-3.8 Gens (GenBank Acc.-No.: M63224) oder der Gerste GerA-Promotor (WO 02/057412) verwendet. Besagte Promotoren sind besonders vorteilhaft, da sie sowohl Mesophyllgewebe-spezifisch als auch pathogeninduzierbar sind. Ferner geeignet ist der Mesophyllgewebe-spezifische Arabidopsis CAB-2 Promotor (GenBank Acc.-No.: X15222), sowie *der Zea mays* PPCZml-Promotor (GenBank Acc.-No.: X63869) oder Homologe davon. Mesophyllgewebe-spezifisch meint eine durch die spezifische Wechselwirkung von in der Promotorsequenz vorhandenen Cis-Elementen und an diese bindende Transkriptionsfaktoren hervorgerufene Beschränkung der Transkription eines Gens auf möglichst wenige, das Mesophyllgewebe enthaltende Pflanzengewebe, vorzugsweise ist eine auf das Mesophyllgewebe beschränkte Transkription gemeint.

Weitere Mesophyll-spezifische Promotoren sind PPCZm1 (=PEPC; Kausch (2001) Plant Mol. Biol. 45: 1-15); OsrbcS (Kyozuka et al. (1993) Plant Phys. 102: 991-1000); OsPPDK, acc. AC099041; TaGF-2.8, acc. M63223 (Schweizer (1999) Plant J. 20: 541-552); TaFBPase, acc. X53957;.TaWIS1, acc. AF467542 (US 2002/115849); HvBIS1, acc. AF467539 (US 2002/115849); ZmMIS1, acc. AF467514 (US 2002/115849); HvPR1a, acc. X74939 (Bryngelsson et al. (1994) Molecular Plant-Microbe Interactions 7(2): 267-75; HvPR1b, acc. X74940 (Bryngelsson et al. (1994) Molecular Plant-Microbe Interactions 7(2): 267-75); HvB1,3gluc; acc. AF479647; HvPrx8, acc. AJ276227 (Kristensen et al (2001) Molecular Plant Pathology 2(6): 311-317; und HvPAL, acc. X97313 (Wei (1998) Plant Molecular Biology 36: 101-112).

### f) Epidermis-spezifische Promotoren

"Epidermisgewebe" oder Epidermis meint die äußeren Gewebeschichten der Pflanzen. Die Epidermis kann ein- bis mehrschichtig sein; es gibt epidermis- "angereicherte" Genexpression, wie z.B. von Cer3 , die als Marker dienen kann (Hannoufa (1996) Plant J. 10 (3): 459-467).

Unter "Epidermis" versteht der Fachmann vorzugsweise das vorherrschende Abschlussgewebe primärer oberirdischer Pflanzenteile, z.B. des Sprosses, der Blätter, Blüten, Früchte und Samen.

Epidermis-spezifische Promotoren sind beispielsweise WIR5 (=GstA1), acc. X56012 (Dudler & Schweizer, unveröff.); GLP4, acc. AJ310534 (Wei (1998) Plant Molecular Biology 36: 101-112); GLP2a, acc. AJ237942 (Schweizer (1999). Plant J 20: 541-552); Prx7, acc. AJ003141 (Kristensen (2001) Molecular Plant Pathology 2(6): 311-317); GerA, acc. AF250933 (Wu (2000) Plant Phys. Biochem. 38: 685-698); OsROC1, acc. AP004656; RTBV, acc. AAV62708, AAV62707 (Klöti (1999) PMB 40: 249-266) und Cer3 (Hannoufa (1996) Plant J. 10 (3): 459-467).

### g) Entwicklungsabhängige Promotoren

Weitere geeignete Promotoren sind beispielsweise fruchtreifungs-spezifische Promotoren, wie beispielsweise der fruchtreifungs-spezifische Promotor aus Tomate (WO 94/21794, EP 409 625). Entwicklungsabhängige Promotoren schließen zum Teil die gewebespezifischen Promotoren ein, da die Ausbildung einzelner Gewebe naturgemäß entwicklungsabhängig erfolgt.

Besonders bevorzugt sind konstitutive, sowie Blatt- und/oder Stengel-spezifische, pathogen-induzierbare, Wurzel-spezifische, Mesophyllgewebe-spezifische Promotoren, wobei konstitutive, pathogen-induzierbare, Mesophyllgewebe-spezifische und Wurzel-spezifische Promotoren am meisten bevorzugt sind.

Es können ferner weitere Promotoren operativ mit der zu exprimierenden Nukleinsäuresequenz verknüpft sein, die eine Expression in weiteren Pflanzengeweben oder in anderen Organismen, wie zum Beispiel *E.coli*-Bakterien ermöglichen. Als Pflanzen Promotoren kommen im Prinzip alle oben beschriebenen Promotoren in Frage.

Weitere zur Expression in Pflanzen geeignete Promotoren sind beschrieben (Rogers et al. (1987) Meth. in Enzymol. 153: 253-277; Schardl et al. (1987) Gene 61: 1-11; Berger et al. (1989) Proc. Natl. Acad. Sci. USA 86: 8402-8406).

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien z. B. weitere epidermis-spezifische regulatorische Nukleinsäureelemente identifizieren. Dabei wird z.B. in einem ersten Schritt das gewünschte Gewebe aus dem gewünschten Organismus, aus dem die regulatorischen Sequenzen isoliert werden sollen, isoliert und daraus die gesamte poly(A)⁺-RNA isoliert und eine cDNA-Bank angelegt. In einem zweiten Schritt werden mit Hilfe von cDNA-Klonen, die auf poly(A)⁺-RNA-Molekülen aus einem anderen Gewebe basieren, aus der ersten Bank mittels Hybridisierung diejenigen Klone identifiziert, deren korrespondierende poly(A)⁺-RNA-Moleküle lediglich im gewünschten Gewebe akkumulieren. Anschließend werden mit Hilfe dieser so identifizierten cDNAs Promotoren isoliert, die über Gewebe-spezifische regulatorische Elemente verfügen. Dem Fachmann stehen darüber hinaus weitere auf PCR basierende Methoden für die Isolierung geeigneter Gewebe-spezifischer Promotoren zur Verfügung.

Die in den erfindungsgemäßen Expressionskassetten oder Vektoren enthaltenen Nukleinsäuresequenzen können mit weiteren genetischen Kontrollsequenzen neben einem Promotor operativ verknüpft sein. Der Begriff der genetischen Kontrollsequenzen ist breit zu verstehen und meint all solche Sequenzen, die einen Einfluss auf das Zustandekommen oder die Funktion des erfindungsgemäßen rekombinanten Nukleinsäuremoleküls haben. Genetische Kontrollsequenzen modifizieren zum Beispiel die Transkription und Translation in prokaryotischen oder eukaryotischen Organismen. Vorzugsweise umfassen die erfindungsgemäßen Expressionskassetten 5'-stromaufwärts von der jeweiligen transgen zu exprimierenden Nukleinsäuresequenz einen Promotor mit einer vorab beschriebenenen Spezifität und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH (1991) J. Biol. Chem. 266(26): 17131 -17135) und Hitzestress (Schoffl F et al. (1989) Mol. Gen. Genet. 217(2-3): 246-53) beschrieben.

Prinzipiell können alle natürlichen Promotoren mit ihren Regulationssequenzen wie die oben genannten für das erfindungsgemäße Verfahren verwendet werden. Darüber hinaus können auch synthetische Promotoren vorteilhaft verwendet werden.

Genetische Kontrollsequenzen umfassen ferner auch die 5'-untranslatierten Regionen, Introns oder die nicht-kodierende 3'-Region von Genen wie beispielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Rolle bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl. Acids Res. 15: 8693-8711) und dergleichen. Sie können ferner die Gewebespezifität fördern (Rouster J et al. (1998) Plant J. 15: 435-440).

Das rekombinante Nukleinsäuremolekül kann vorteilhafterweise eine oder mehrere sogenannte "enhancer"-Sequenzen funktionell verknüpft mit dem Promotor enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignalen aus *Agrobacterium tumefaciens,* insbesondere des Gens 3 der T-DNA (Octopin Synthase) des Ti-Plasmids pTiACHS entsprechen (Gielen et al. (1984) EMBO J. 3: 835 ff) oder funktionelle Äquivalente davon. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promotor eines bestimmten Gens gegen einen Promotor mit Spezifität für die embryonale Epidermis und/oder die Blüte ausgetauscht werden.

Ein rekombinantes Nukleinsäuremolekül und ein von ihr abgeleiteter Vektor können weitere Funktionselemente enthalten. Der Begriff Funktionselement ist breit zu verstehen und meint all solche Elemente, die einen Einfluss auf Herstellung, Vermehrung oder Funktion der erfindungsgemäßen Nukleinsäuremoleküle, Vektoren oder transgenen Organismen haben. Beispielhaft aber nicht einschränkend seien zu nennen:
a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin verleihen. Besonders bevorzugte Selektionsmarker sind solche, die eine Resistenz gegen Herbizide verleihen. Beispielhaft seien genannt: DNA-Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP-Synthasegene), die eine Resistenz gegen Glyphosat^{®} (N-(phosphonomethyl)glycin) verleihen, das für Glyphosat^{®} degradierende Enzyme kodierende gox-Gen (Glyphosatoxidoreduktase), das deh-Gen (kodierend für eine Dehalogenase, die Dalapon inaktiviert), Sulfonylharnstoff- und Imidazolinon inaktivierende Acetolactatsynthasen sowie bxn-Gene, die für Bromoxynil degradierende Nitrilaseenzyme kodieren, das aasa-Gen, das eine Resistenz gegen das Antibiotikum Apectinomycin verleiht, das Streptomycinphosphotransferase (SPT)-Gen, das eine Resistenz gegen Streptomycin vermittelt, das Neomycinphosphotransferase (NPTII)-Gen, das eine Resistenz gegen Kanamycin oder Geneticidin verleiht, das Hygromycinphosphotransferase (HPT)-Gen, das eine Resistenz gegen Hygromycin vermittelt, das Acetolactatsynthase-Gen (ALS), das eine Resistenz gegen Sulfonylharnstoff-Herbizide verleiht (z.B. mutierte ALS-Varianten mit z.B. der S4 und/oder Hra Mutation).
b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn und Groskreutz (1999) Mol. Biotechnol. 13(1): 29-44) wie das "green fluorescence protein" (GFP) (Sheen et al. (1995) Plant Journal 8(5): 777-784; Haseloff et al. (1997) Proc. Natl. Acad. Sci. USA 94(6): 2122-2127; Reichel et al.(1996) Proc. Natl. Acad. Sci. USA 93(12): 5888-5893; Tian et al. (1997) Plant Cell Rep. 16: 267-271; WO 97/41228; Chui et al. (1996) Curr Biol 6: 325-330; Leffel et al. (1997) Biotechniques 23(5): 912-8), die Chloramphenicoltransferase, eine Luziferase (Ow et al. (1986) Science 234: 856-859; Millar et al. (1992) Plant Mol. Biol. Rep. 10: 324-414), das Aequoringen (Prasher et al. (1985) Biochem. Biophys. Res. Commun. 126(3): 1259-1268), die β-Galaktosidase, R-Locus Gen (kodiert ein Protein, das die Produktion von Anthocyaninpigmenten (rote Färbung) in pflanzlichen Gewebe reguliert und so eine direkte Analyse der Promotoraktivität ohne Zugabe zusätzlicher Hilfstoffe oder chromogener Substrate ermöglicht; Dellaporta et al., In: Chromosome Structure and Function: Impact of New Concepts, 18th Stadler Genetics Symposium, 11:263-282, 1988), ganz besonders bevorzugt ist die ß-Glukuronidase (Jefferson et al. (1987) EMBO J. 6: 3901-3907).
c) Replikationsursprünge, die eine Vermehrung der erfindungsgemäßen Expressionskassetten oder Vektoren in zum Beispiel *E.coli* gewährleisten. Beispielhaft seien genannt ORI (origin of DNA replication), der pBR322 ori oder der P15A ori (Sambrook und Russell, *vide supra*).
d) Elemente, die für eine Agrobakterium-vermittelte Pflanzentransformation erforderlich sind, wie zum Beispiel die rechte oder linke Begrenzung der T-DNA oder die vir-Region.

Zur Selektion erfolgreich transformierter Zellen ist es in der Regel erforderlich, einen selektionierbaren Marker zusätzlich einzuführen, der den erfolgreich transformierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. und damit die Selektion der transformierten Zellen gegenüber untransformierten erlaubt (McCormick et al. (1986) Plant Cell Reports 5: 81-84).

Die vorliegende Erfindung betrifft weiterhin transgene Pflanzenzellen und transgene Pflanzen, die eine beschriebene Nukleinsäuresequenz oder ein erfindungsgemäßes rekombinantes Nukleinsäuremolekül umfassen, sowie Teile der Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanze.

Bevorzugt handelt es sich dabei um Pflanzen, die zu der Familie der Poaceae gehören, besonders bevorzugt sind Pflanzen ausgewählt aus den Pflanzengattungen Hordeum, Avena, Secale, Triticum, Sorghum, Zea, Saccharum und Oryza, ganz besonders bevorzugt Pflanzen ausgewählt aus den Arten *Hordeum vulgare* (Gerste), *Triticum aestivum* (Weizen), *Triticum aestivum subsp.spelta* (Dinkel), Triticale, *Avena sativa* (Hafer), *Secale cereale* (Roggen), *Sorghum bicolor* (Hirse), *Zea mays* (Mais), *Saccharum officinarum* (Zuckerrohr) und *Oryza sativa* (Reis).

Das Verfahren gemäß vorliegender Erfindung eignet sich aber auch für dikotyle Nutzpflanzen wie beispielsweise Baumwolle, Leguminosen wie Hülsenfrüchte und insbesondere Alfalfa, Sojabohne, Raps, Tomate, Zuckerrübe, Kartoffel, Sonnenblume, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitalis umfassen. Besonders bevorzugt sind die dikotylen Pflanzen Zuckerrübe, Raps, Soja, Tomate, Kartoffel und Tabak. Weitere Nutzpflanzen können dem US-Patent Nr. 6,137,030 entnommen werden.

Die spezifische Expression des beschriebenen Proteins in den erfindungsgemäßen Pflanzen bzw. in den erfindungsgemäßen Pflanzenzellen kann mit Hilfe herkömmlicher molekularbiologischer und biochemischer Methoden nachgewiesen und verfolgt werden. Dem Fachmann sind diese Techniken bekannt und er ist problemlos in der Lage, eine geeignete Nachweismethode zu wählen, beispielsweise eine Northem-Blot-Analyse zum Nachweis Protein-spezifischer RNA bzw. zur Bestimmung der Höhe der Akkumulation von Protein-spezifischer RNA oder eine Southern-Blot- bzw. PCR-Analyse zum Nachweis von für ein beschriebenes Protein kodierenden DNA-Sequenzen. Die dazu verwendeten Sonden- bzw. Primersequenzen können zu der in SEQ ID No. 1 angegebenen Sequenz entweder identisch sein oder einige wenige Abweichungen von dieser Sequenz aufweisen.

Selbstverständlich kann das erfindungsgemäße Verfahren auch mit anderen Verfahren zur Erhöhung der Pathogenresistenz in transgenen Pflanzen kombiniert werden. So kann beispielsweise die Polypeptidmenge, die Aktivität oder die Funktion eines oder mehrerer Resistenzfaktoren ausgewählt aus der Gruppe bestehend aus Bax Inhibitor 1-Protein aus *Hordeum vulgare* (GenBank Acc.-No.: AJ290421), aus *Nicotiana tabacum* (GenBank Acc.-No.: AF390556), Reis (GenBank Acc.-No.: AB025926), Arabidopsis (GenBank Acc.-No.: AB025927) bzw. Tabak und Raps (GenBank Acc.-No.: AF390555, Bolduc et al. (2003) Planta 216: 377-386), ROR2 (z.B. aus Gerste (GenBank Acc.-No.: AY246906)), SnAP34 (z.B. aus Gerste (GenBank Acc.-No.: AY247208)) und/oder Lumenal Binding Protein BiP z.B. aus Reis (GenBank Acc.-No. AF006825) durch geeignete Verfahren gesteigert werden. Ebenso kann die Polypeptidmenge, die Aktivität oder die Funktion eines oder mehrerer Resistenzfaktoren ausgewählt aus der Gruppe bestehend aus RacB (z.B. aus Gerste (GenBank Acc.-No.: AJ344223)), CSL1 (z.B. aus Arabidopsis (GenBank Acc.-No.: NM116593)), HvNaOX (z.B. aus Gerste (GenBank Acc.-No.: AJ251717); EP 1 525 315), MLO (z.B. aus Gerste (GenBank Acc.-No. Z83834); WO 98/04586, WO 00/01722, WO 99/47552), ARM1 (armadillo repeat protein; EP-Anmeldenummer 05110468.5) durch geeignete Verfahren vermindert werden.

Außerdem offenbart ist die Verwendung der erfindungsgemäßen transgenen Organismen und der von ihnen abgeleiteten Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien.

Weiterhin offenbart ist ein Verfahren zur rekombinanten Herstellung von Pharmazeutika oder Feinchemikalien in Wirtsorganismen, wobei ein Wirtsorganismus oder ein Teil davon mit einem der oben beschriebenen rekombinanten Nukleinsäuremoleküle transformiert wird und dieses Nukleinsäuremolekül ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakzinen ist beschrieben bei Hood und Jilka (1999). Curr. Opin. Biotechnol.10(4): 382-6; Ma und Vine (1999) Curr. Top. Microbiol. Immunol. 236: 275-92.

Die Identifizierung des leucine-rich-repeat-enthaltenden Proteins mit einer Kinasedomäne, RNR8, aus Gerste als Gen, das eine Resistenz der Gerste gegenüber Isolaten von *Blumeria graminis* vermittelt, und seine Anwendung zur Vermittlung der Pathogenresistenz in transgenen Pflanzen bzw. Pflanzenzellen wird nun im Folgenden dargestellt.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose- und Nylon-Membranen, Verknüpfen von DNA-Fragmenten, Transformation von E. coli-Zellen, Anzucht von Bakterien und die Sequenzanalyse rekombinanter DNA wurden wie bei Sambrook et al. (2001), *vide supra,* beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA 74: 5463-5467).

### Beispiel 3: BAC-Screening zur Identifizierung des Klons, der die erfindungsgemäße Sequenz enthält

Für die Identifizierung des Genes kodierend für die erfindungsgemäße Sequenz in Gerste wurden DNA-Pools aus einer Gersten-BAC-Bibliothek (Yu et. al. (2000) TAG 101: 1093-99) verwendet. BAC-Klone, welche die erfindungsgemäße Sequenz tragen, wurden mittels PCR unter Verwendung der Primer 5' CTT TCG TGC TTA TGT GGG TGT GAC (SEQ ID NO: 4) und 5' CAT GAG GAG TCT GCA ATA AGG A (SEQ ID NO: 5) identifiziert.

Die PCR wurde aufgrund ihrer hohen Sensitivität zum Nachweis der gesuchten DNA-Sequenz gewählt. Die Analyse wurde in 20 µl Reaktionsvolumen durchgeführt. Der Reaktionsansatz bestand aus 10 mM Tris-HCl, pH 9,0; 50 mM KCI; 0,1 % Triton X-1 00, 0,2 mM dNTP; 2 mM MgCl₂, je 0,6 µM Oligonukleotide und Taq-Polymerase (Konzentration im Reaktionsansatz: ∼ 1U µl⁻¹). Pro Reaktionsansatz wurden entweder 10 ng BAC-Pool-DNA oder 2 µl Bakterienkultur (für Kolonie-PCR) verwendet. Vorhandene cDNA-Sequenzen dienten als Grundlage zur Ableitung der Oligonukleotide 5'GGA TTT GTC ACG TCC AAC CT (SEQ ID NO: 6) und 5'ATT GGC AAT TGT GAT AGC CC (SEQ ID NO: 7).

Die zu amplifizierende BAC-DNA und die Primer wurden vorgelegt und anschließend mit dem PCR-Reaktionsansatz vermischt. Zum Abtöten und Aufschließen der Bakterien in einer Kolonie-PCR wurde die Vorlage vor der Zugabe des PCR-Reaktionsgemisches für 5 min auf 95°C erhitzt. Zur Denaturierung der doppelsträngigen DNA wurde ein initialer Schritt von 5 min bei 95°C benutzt. Die Touch-Down PCR-Reaktion erfolgte in den Abschnitten 30 s 95°C; 30 s 60 nach 55°C und 60 s 72°C für die ersten 10 Zyklen. Dabei wurde mit jedem Zyklus die Temperatur um 0,5 °C (60 nach 55°C) gesenkt. Weitere 30 Zyklen erfolgten in den Abschnitten 30 s 95°C; 30 s 55°C und 60 s 72°C. Zur abschließenden Kettenverlängerung wurde 5 min bei 72°C inkubiert, bevor die Reaktion auf eine Temperatur von 20°C abgekühlt und konstant gehalten wurde. Die Analyse der PCR-Experimente erfolgte aufgrund des erwartet kurzen Reaktionsproduktes von 189 bp mit 2,5 %igen Agarosegelen in 0,5x TBE-Puffer.

Identifizierte Einzelklone wurden für die Gen- und Promotoridentifizierung in zwei Stufen subkloniert. Zunächst wurde die BAC-DNA eines Einzelklones mittels Qiagen-Säule isoliert (Maxi-Kit; Qiagen; Isolierung nach Herstellerprotokoll). Durch Scherung (Hydroshear: Genomic Solutions) wurden von dieser BAC-DNA 5 - 10 kbp Fragmente erzeugt und die entstandenen Enden mit Klenow zu glatten Enden aufgefüllt (Reaktion nach Protokoll des Herstellers). Die Selektion der Fragmentlängen erfolgte über ein 0,8%iges Agarosegel in 0,5 % TBE. Aus dem Gel wurde der entsprechende Fragmentlängenbereich herausgeschnitten und die DNA aus dem Agarosegel mit Hilfe von Qiagen Gel Extraction Kit eluiert (Elution nach Protokoll des Herstellers). Die eluierten 5-10 kbp Fragmente wurden in einen mit EcoRV linearisierten pBluescript II SK(-) Vektor mit glatten dephosphorylierten Enden ligiert (Restriktion und Dephosphorylierung entsprechend den Herstellerangaben) und in hochkompetente *E. coli* Zellen chemisch-thermisch transformiert. Anschließend wurden die Transformanten mit Hilfe eines Picking-Roboters (Qpick, Genetix) willkürlich geordnet und in Mikrotiterplatten mit LB-Medium überführt.

Mittels PCR wurde dasjenige Subfragment ausgewählt, welches das interessierende Gen trägt und die Länge des potenziellen 5'-upstream Bereichs maximiert. Das gewählte Subfragment wurde erneut in 1-2 kbp Fragmente geschert, ligiert, transformiert und die Klone in Microtiterplatten gelagert (s.o.). Von den gepickten Klonen wurden 96 Kolonien willkürlich ausgewählt und mit dem TempliPhi Protokoll nach Herstellerprotokoll sequenziert. Die Sequenzen wurden assembliert. Die erhaltene Sequenzinformation wurde für die Annotation der kodierenden Exons im Vergleich zu bereits bekannten Sequenzen anderer Organismen genutzt, um die erfindungsgemäße Sequenz und ihre potenziellen Promotoren zu bestimmen.

### Beispiel 4: Subklonierung des RNR8-Gens in pIPKTA9

Um genauer zu überprüfen, ob das identifizierte Gen RNR8 für die Vermittlung der Pathogenresistenz verantwortlich ist, wurde dieses Gen anhand des folgenden Protokolls in den Vektor pIPKTA9 subkloniert:
1. Verdau NBS-LRR BAC (Nr. 027N11) mit *Psp* 1406 I und *Xma*JI. Dies schneidet fast den kompletten kodierenden Teil des RNR8-Gens heraus.
2. Blunt ending Enden mit Klenow.
3. Gelelution der 11 kb Bande von Schritt 2 mit dem Qiagen Gelextraktionskit.
4. Ligation der 11 kb Bande NBS_LRR (Schritt 3) in TA38 in Anwesenheit von *Swa* I (Douchkov et al. (2005) Molecular Plant-Microbe Interactions 18: 755-761.)
5. PCR eines NBS-LRR 1 kb Fragmentes, um den kodierenden Bereich des Gens am 5'-Ende (upstream) zu komplettieren. Thermal Ace Polymerase (produziert Fragmente mit blunt ends).
   Primer NBS-LRR F1: GCT GAA CCA ACC CGG GGA GAA ATA (SEQ ID NO: 8)
   Primer NBS-LRR F1: AGA TGA TCG GAA GAA CAG TGC AAC (SEQ ID NO: 9)
6. Purification PCR (Schritt 5) mit MinElute Platten.
7. Klonierung des 5' NBS-LRR PCR Fragments (Schritt 6) in pIPKTA9, geschnitten durch *Sma* I.
8. Verdau des 11kb LRR in TA38 (Schritt 4) mit *Aar* I und *Not* I.
9. Gelelution des 11 kb LRR von Schritt 8.
10. Verdau von pIPKTA9_NBS_LRR_5' (Schritt 7) mit *Aar I* und *Not I*
11. Gelelution - 3,5 kb Vektor Bande von Schritt 10
12. Ligation der Fragmente von Schritt 7 und 11 mit T4 DNA Ligase (Fermentas).
13. Verifikation des finalen Konstrukts pIPKTA9_NBS-LRR durch Sequenzierung.

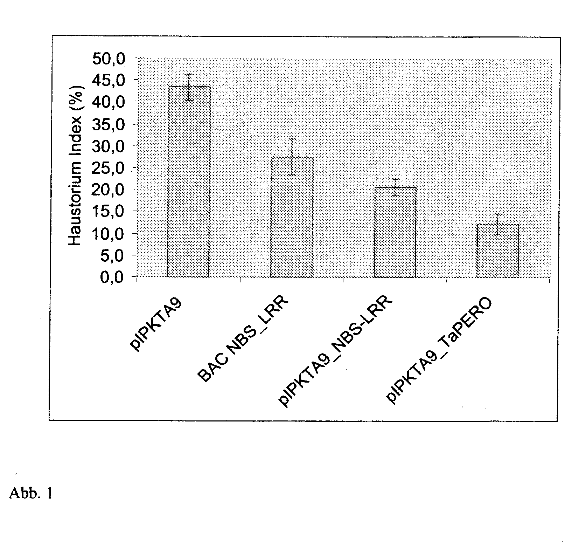

Da mit dem Konstrukt pIPKTA9_NBS-LRR dieselben Effekte erzielt wurden wie mit dem BAC-Klon, konnte daraus geschlossen werden, dass RNR8 das für die beobachtete Pathogenresistenz verantwortliche Gen auf BAC Nr. 027N11 ist (siehe hierzu auch Abbildung 1).

### Beispiel 5: Transiente Expression in Weizen durch Partikelbeschuss

Folgendes Gemisch von Konstrukten wurde mit einer "Genkanone" (Bio-Rad, Modell PDS-1000/He, Hepta-Adapter) mittels biolistischer Transformation gemäss der Methode von Douchkov et al. (2005) Mol. Plant-Microbe Interact. 18: 755-761 in Weizenblätter eingebracht:

| Plasmid | Ansatz 1 | Ansatz 2 | Ansatz 3 | Ansatz 4 |
|---|---|---|---|---|
| pUbiGUS (Reportergenkonstrukt) | 7 µg/Schuss | 7 µg/Schuss | 7 µg/Schuss | 7 µg/Schuss |
| pIPKTA9 (leerer Überexpressionsvektor) | 7 µg/Schuss | --- | --- | --- |
| pIPKTA9_TaPERO | --- | | 7 µg/Schuss | --- |
| BAC 027N11 | --- | 14 µg/Schuss | | --- |
| pIPKTA9_NBS LRR | --- | --- | --- | 7 µg/Schuss |

Für die Beschichtung mit DNA wurden pro Schuss 2.18 mg Goldpartikel (1.0 µm Durchmesser, Partikeldichte 25 mg ml⁻¹ in 50% (v/v) Glyzerin) mit 14-21 µg "supercoiled" DNA gemischt und mit 1 M Ca(NO₃)₂ pH 10 versetzt, so dass die Endkonzentration von Ca(NO₃)₂ 0.5 M betrug. Nach Zentrifugieren und Waschen mit 70 % (v/v) Ethanol wurden die Partikel in 96 % (v/v) Ethanol resuspendiert und auf den 7 Makrocarriern verteilt. Die Partikel wurden in einem Vakuum (3.6x10³ Pa) durch eine Heliumdruckwelle von 7.6x10⁶ Pa auf jeweils 7 Blattsegmente von 7 Tage alten Weizenpflanzen (Sorte Kanzler) eingebracht. Zum Beschuss wurden die Blattsegmente in eine Petrischale auf 0.5% (w/v) Phytoagar gelegt, der mit 20 µg ml⁻¹ Benzimidazol versetzt war. Die Blätter wurden anschließend bei +20°C und indirektem Tageslicht für 4 h inkubiert.

### Beispiel 6: Inokulation der Blattsegmente

Die bombardierten Blätter wurden auf 1 % (w/v) Phytoagar mit 20 µg ml⁻¹ Benzimidazol in 20 x 20 cm großen Polycarbonat-Schalen umgelegt. In einem Inokulationsturm erfolgte die Infektion mit Weizenmehltau-Sporen, indem Sporen von stark infizierten Weizenblättern in den Turm geschüttelt wurden. Die Inokulumdichte lag bei rund 200 Sporen/mm². Nach 5 min wurden die Schalen entfernt, geschlossen und bei +20°C und indirektem Tageslicht für 40-48 h inkubiert.

### Beispiel 7: Histochemischer GUS-Nachweis

Die Blätter wurden unter Vakuum mit der GUS-Nachweislösung (10 mM EDTA, 1.4 mM K₃[Fe(CN)₆], 1.4 mM K₄[Fe(CN)₆], 0.1 % (v/v) Triton X-100, 20 % (v/v) Methanol, 1 mg/ml 3-Bromo-4-chloro-3-indolyl-β-D-glucuronsäure, 100 mM Na-Phosphatpuffer, pH 7.0) infiltriert und über Nacht bei +37°C inkubiert. Nach Entfernen der Nachweislösung wurden die Blätter mit einer Lösung aus 7.5 % (w/v) Trichloressigsäure und 50 % (v/v) Methanol für 15 min bei +20°C entfärbt.

Die Lichtmikroskopie erfolgte mit einem Zeiss Axiolab-Mikroskop bei 200-facher Vergrößerung. Zellen mit GUS-Expression besitzen einen blau gefärbten Zellinhalt. Mittels quantitativer Mikroskopie wurden pro Schuss die Anzahl GUS-gefärbter Zellen und die Anzahl der GUS-gefärbten Zellen, die mindestens 1 Haustorium des Weizenmehltaupilzes tragen, gezählt. Der Suszeptibilitätsindex berechnete sich aus der Anzahl der haustoriumtragenden GUS-pos. Zellen/Alle GUS-positiven Zellen.

Das Ergebnis des Inokulationsexperiments ist in Abbildung 1 dargestellt. Es zeigte sich, dass die Expression des erfindungsgemäßen Proteins vom BAC-Klon oder dem Expressionsvektor pIPKTA9_NBS-LRR die Suszeptibilität der Weizenpflanzen gegenüber Weizenmehltau im Vergleich zu Pflanzen, die mit dem Leervektor transformiert worden waren, um etwa 50% reduziert.

### Abbildungen

Abbildung 1: Transientes Komplementationsexperiment in Weizen mit Gersten-BAC-Klon 027N11 (Morex BAC-Bank), der die leucine-rich-repeat Proteinkinase Rnr8 enthält. Weizenblätter wurden ko-bombardiert mit pUbiGUS und entweder pIPKTA9 (leerer Überexpressionsvektor), BAC-Klon 027N11, pIPKTA9_NBS LRR (siehe Beispiel 4) oder pIPKTA9:TaPERO (Überexpressionskonstrukt für eine Peroxidase als Positivkontrolle). Dargestellt sind Mittelwerte und Standardabweichungen aus 2 unabhängigen Versuchen mit je 2 Parallelbombardments.

## Patentansprüche

1. Verfahren zur Erhöhung der Pilzpathogenresistenz in transgenen Pflanzen, **dadurch gekennzeichnet, dass** eine DNA-Sequenz ausgewählt aus der Gruppe bestehend aus:
i) Nukleinsäuresequenzen umfassend Nukleotidsequenzen, die den kodierenden Sequenzen von SEQ ID No. 1 entsprechen,
ii) Nukleinsäuresequenzen umfassend Nukleotidsequenzen, die für ein Protein mit der in SEQ ID No. 2 angegebenen Aminosäuresequenz kodieren,
iii) Nukleinsäuresequenzen umfassend Nukleotidsequenzen, die zu den kodierenden Sequenzen von SEQ ID No. 1 eine Sequenzidentität von mindestens 90% über die jeweils gesamte Sequenzlänge aufweisen, und/oder
iv) Nukleinsäuresequenzen umfassend Nukleotidsequenzen, die unter stringenten Bedingungen mit einem komplementären Strang einer Nukleotidsequenz von i) bis iii) hybridisieren,
die für ein Protein kodiert, welches in Pflanzen eine erhöhte Pilzpathogenresistenz vermittelt, in eine Pflanze bzw. Pflanzenzelle eingeführt und dort exprimiert wird, wobei das Pathogen Mehltau ist.

2. Verfahren nach Anspruch 1,
**dadurch gekennzeichnet, dass** es die folgenden Schritte umfasst:
a) Herstellen eines rekombinanten Nukleinsäuremoleküls umfassend die folgenden Elemente in 5'-3'-Orientierung:
- regulatorische Sequenzen eines in Pflanzenzellen aktiven Promotors,
- operativ daran gebunden eine DNA-Sequenz wie in Anspruch 1 definiert,
- ggf. operativ daran gebunden regulatorische Sequenzen, die in der Pflanzenzelle als Transkriptions-, Terminations- und/oder Polyadenylierungssignale dienen können,
b) Übertragen des rekombinanten Nukleinsäuremoleküls aus a) in Pflanzenzellen und gegebenenfalls Integration ins pflanzliche Genom, und
c) Regenerieren von Pflanzen aus den transformierten Pflanzenzellen.

3. Verfahren nach Anspruch 2,
**dadurch gekennzeichnet, dass** die DNA-Sequenz unter der Kontrolle eines konstitutiven Promotors, bevorzugt dem 35S CaMV- oder Ubiquitinpromotor, exprimiert wird.

4. Rekombinantes Nukleinsäuremolekül wie in Anspruch 2 definiert,
**dadurch gekennzeichnet, dass** die DNA-Sequenz unter der Kontrolle eines gewebespezifischen Promotors exprimiert wird.

5. Rekombinantes Nukleinsäuremolekül nach Anspruch 4,
**dadurch gekennzeichnet, dass** der gewebespezifische Promotor ein Epidermis-, Mesophyll-oder Blattspezifischer Promotor ist.

6. Rekombinantes Nukleinsäuremolekül wie in Anspruch 2 definiert,
**dadurch gekennzeichnet, dass** die DNA-Sequenz unter der Kontrolle eines induzierbaren Promotors exprimiert wird.

7. Rekombinantes Nukleinsäuremolekül nach Anspruch 6,
**dadurch gekennzeichnet, dass** der induzierbare Promotor ein pathogen- oder wundinduzierbarer Promotor ist.

8. Transgene Pflanzenzelle, umfassend eine transgene Nukleinsäuresequenz wie in Anspruch 1 definiert oder ein rekombinantes Nukleinsäuremolekül wie in einem der Ansprüche 2 bis 7 definiert, die eine gegenüber Wildtypzellen erhöhte Resistenz gegenüber Mehltau aufweist.

9. Transgene Pflanzenzelle nach Anspruch 8, die eine erhöhte Resistenz gegenüber Formae speciales des Mehltaus aufweist.

10. Transgene Pflanze, umfassend eine Pflanzenzelle nach einem der Ansprüche 8 oder 9.

11. Transgene Pflanze nach Anspruch 10,
**dadurch gekennzeichnet, dass** es sich bei der transgenen Pflanze um eine monokotyledone Pflanze, bevorzugt um eine Pflanze, die zu den Gattungen Avena, Triticum, Secale, Hordeum, Oryza, Panicum, Pennisetum, Setaria, Sorghum, Zea und dergleichen gehört, handelt.

12. Transgene Pflanze nach Anspruch 10,
**dadurch gekennzeichnet, dass** es sich bei der transgenen Pflanze um eine dikotyledone Pflanze, bevorzugt um Baumwolle, Leguminosen wie Hülsenfrüchte und insbesondere Alfalfa, Sojabohne, Raps, Canola, Tomate, Zuckerrübe, Kartoffel, Zierpflanzen, Sonnenblume, Tabak sowie Bäume handelt.

## Claims

1. A method of increasing the resistance to fungal pathogens in transgenic plants, **characterized in that** a DNA sequence selected from the group consisting of:
i) nucleic acid sequences comprising nucleotide sequences which correspond to the coding sequences of SEQ ID No. 1,
ii) nucleic acid sequences comprising nucleotide sequences which code for a protein with the amino acid sequence shown in SEQ ID No. 2,
iii) nucleic acid sequences comprising nucleotide sequences which have at least 90% sequence identity with the coding sequences of SEQ ID No. 1 over the total sequence length in each case, and/or
iv) nucleic acid sequences comprising nucleotide sequences which, under stringent conditions, hybridize with a complementary strand of a nucleotide sequence of i) to iii),
which codes for a protein which confers an increased resistance to fungal pathogens in plants, is introduced into, and expressed in, a plant or plant cell, the pathogen being mildew.

2. The method as claimed in claim 1, **characterized in that** it comprises the following steps:
a) generating a recombinant nucleic acid molecule comprising the following elements in 5'-3' orientation:
- regulatory sequences of a promoter which is active in plant cells,
- a DNA sequence as defined in claim 1 in operable linkage thereto,
- if appropriate, regulatory sequences which may act as transcription, termination and/or polyadenylation signals in the plant cell, in operable linkage thereto,
b) transferring the recombinant nucleic acid molecule from a) into plant cells and, if appropriate, integrating it into the plant genome, and
c) regenerating plants from the transformed plant cells.

3. The method as claimed in claim 2,
**characterized in that** the DNA sequence is expressed under the control of a constitutive promoter, preferably the 35S CaMV or ubiquitine promoter.

4. The recombinant nucleic acid molecule as defined in claim 2,
**characterized in that** the DNA sequence is expressed under the control of a tissue-specific promoter.

5. The recombinant nucleic acid molecule as claimed in claim 4,
**characterized in that** the tissue-specific promoter is an epidermis-, mesophyll- or leaf-specific promoter.

6. The recombinant nucleic acid molecule as defined in claim 2,
**characterized in that** the DNA sequence is expressed under the control of an inducible promoter.

7. The recombinant nucleic acid molecule as claimed in claim 6,
**characterized in that** the inducible promoter is a pathogen- or wound-inducible promoter.

8. A transgenic plant cell, comprising a transgenic nucleic acid sequence as defined in claim 1 or a recombinant nucleic acid molecule as defined in any of claims 2 to 7, which features a resistance to mildew which is increased in comparison with wild-type cells.

9. The transgenic plant cell as claimed in claim 8, which features an increased resistance to Formae speciales of mildew.

10. A transgenic plant, comprising a plant cell as claimed in either of claims 8 and 9.

11. The transgenic plant as claimed in claim 10,
**characterized in that** the transgenic plant is a monocotyledonous plant, preferably a plant which belongs to the genera Avena, Triticum, Secale, Hordeum, Oryza, Panicum, Pennisetum, Setaria, Sorghum, Zea and the like.

12. The transgenic plant as claimed in claim 10,
**characterized in that** the transgenic plant is a dicotyledonous plant, preferably cotton, leguminoses such as pulses and in particular alfalfa, soybean, oilseed rape, canola, tomato, sugar beet, potato, ornamentals, sunflower, tobacco and trees.

## Revendications

1. Procédé pour augmenter la résistance aux pathogènes fongiques dans des plantes transgéniques, **caractérisé en ce qu'**une séquence d'ADN choisie dans le groupe consistant en :
i) des séquences d'acides nucléiques comprenant les séquences nucléotidiques qui correspondent aux séquences codantes de SEQ ID NO:1,
ii) des séquences d'acides nucléiques comprenant les séquences nucléotidiques qui codent pour une protéine ayant la séquence d'acides aminés indiquée dans SEQ ID NO:2,
iii) des séquences d'acides nucléiques comprenant les séquences nucléotidiques qui avec les séquences codantes de SEQ ID NO:1 présentent une identité de séquence d'au moins 90 % sur la totalité de la longueur de la séquence correspondante, et/ou
iv) des séquences d'acides nucléiques comprenant les séquences nucléotidiques qui s'hybrident dans des conditions stringentes à un brin complémentaire d'une séquence nucléotidique de i) à iii),
qui code pour une protéine qui confère à des plantes une résistance accrue aux pathogènes fongiques, est introduite dans une plante ou une cellule végétale et y est exprimée, le pathogène étant le mildiou.

2. Procédé selon la revendication 1, **caractérisé en ce qu'**il comprend les étapes suivantes :
a) fabrication d'une molécule d'acide nucléique recombinante comprenant les éléments suivants selon l'orientation 5'-3':
- des séquences régulatrices d'un promoteur actif dans les cellules végétales,
- une séquence d'ADN telle que définie dans la revendication 1, qui y est fonctionnellement liée,
- des séquences régulatrices qui peuvent servir de signaux de transcription, de terminaison et/ou de polyadénylation dans la cellule végétale, qui y sont éventuellement fonctionnellement liées,
b) le transfert de la molécule d'acide nucléique recombinante de a) dans des cellules végétales, et éventuellement son intégration dans le génome végétal,
et
c) la régénération de plantes à partir des cellules végétales transformées.

3. Procédé selon la revendication 2, **caractérisé en ce que** la séquence d'ADN est exprimée sous le contrôle d'un promoteur constitutif, de préférence le promoteur 35S du CaMV ou le promoteur de l'ubiquitine.

4. Molécule d'acide nucléique recombinante telle que définie dans la revendication 2, **caractérisée en ce que** la séquence d'ADN est exprimée sous le contrôle d'un promoteur spécifique de tissu.

5. Molécule d'acide nucléique recombinante selon la revendication 4, **caractérisée en ce que** le promoteur spécifique de tissu est un promoteur spécifique d'épiderme, de mésophylle ou de feuille.

6. Molécule d'acide nucléique recombinante telle que définie dans la revendication 2, **caractérisée en ce que** la séquence d'ADN est exprimée sous le contrôle d'un promoteur inductible.

7. Molécule d'acide nucléique recombinante selon la revendication 6, **caractérisée en ce que** le promoteur inductible est un promoteur inductible par un pathogène ou une blessure.

8. Cellule végétale transgénique comprenant une séquence d'acide nucléique transgénique telle que définie dans la revendication 1 ou une molécule d'acide nucléique recombinante telle que définie dans l'une des revendications 2 à 7, qui présente vis-à-vis du mildiou une résistance accrue par comparaison avec les cellules de type sauvage.

9. Cellule végétale transgénique selon la revendication 8, qui présente une résistance accrue à des formes spéciales du mildiou.

10. Plante transgénique comprenant une cellule végétale selon l'une des revendications 8 ou 9.

11. Plante transgénique selon la revendication 10, **caractérisée en ce que**, pour ce qui concerne la plante transgénique, il s'agit d'une plante monocotylédone, de préférence d'une plante qui appartient aux genres Avena, Triticum, Secale, Hordeum, Oryza, Panicum, Pennisetum, Setaria, Sorghum, Zea et analogues.

12. Plante transgénique selon la revendication 10, **caractérisée en ce que**, pour ce qui concerne les plantes transgéniques, il s'agit d'une plante dicotylédone, de préférence le coton, de légumineuses telles que les légumineuses, et en particulier de la luzerne, du soja, de la pomme de terre, des plantes ornementales, du tournesol, du tabac, ainsi que des arbres.


### SEQUENCE LISTING

<110> BASF Plant Science
<120> Verfahren zur Herstellung von transgenen Pflanzen mit erhöhter Pathogenresistenz
<130> B 8372/RN
<150> EP 06 119 815.6
   <151> 2006-08-30
<160> 9
<170> PatentIn version 3.3
<210> 1
   <211> 3277
   <212> DNA
   <213> Hordeum
<220>
   <221> misc_feature
   <223> RNR8 spliced genomic sequence
<400> 1
   gaaagatcct gtacttggac tgcatagaaa ggagccaagt cactccactc atcctctcct     60
   aagagagacg gacctcgccg ctgccattgc gctccacttg gaacttggag ctggaggaga    120
   gatgggggcg cggaggacgc catgcggctg gaggctgctc ttcttccttg ccttgcttct    180
   tgccgaggtc cgccatggct cctcttcatc ttcttcaggg gttaaggcgg gagctgctgc    240
   tgatgccctc cctcctaggc tctcctctgc tgaagtgcac actcttcgcc ggattgcggc    300
   gaatatgggg atatcgcatt ggaacttctc cggcaatccc tgtgaaccca acggcagctt    360
   ggtgtgtgac tgctccttca acaacaatac catatgccat gccactgaga tattcctcaa    420
   ggaacagaac ttcaccggcc agctcccacc agactttgct gatctccctc atctcctcca    480
   gctagaccta agcaggaacg tgttccatgg cacagtgcct gaccggtggg cccggatgag    540
   gctgcaagga ctgtcactaa tgggaaacag attgtcaggg ccttttccca tggctcttac    600
   gaggatcaca accttgacta gcctgagcat tgaagcaaat gagttccgtg ggcaaatccc    660
   agcagaaatt gggcatctca cgcaaataga taagctgata atatcaacca acgagttcac    720
   tggacccctg ccggctgctc tttccttgct gaccaattta accgacttaa ggatttctgg    780
   aaacaattta tctggaaggg ttcctgattt cttggctaaa ctgacaaagc ttgcaaaact    840
   gcaaatcgaa ggatctttgc tggaagggcc tattcccctg ggcttatcca aattgacaaa    900
   cctttctgat ctgaggatta gtgatctgag aggcagtgga tcagctttcc cggatctaag    960
   cagaatgcca tcgatgaaaa cattagtcct caggaattgt tcaatcagcg gaggcatccc   1020
   ttcttacata tgggtcatgg aaaatcttaa gcatctggat ctgagcttta atgaactgac   1080
   tggaaaagta tcagattcga tcactcttat gggaagcgta gattacatct atctaactgg   1140
   aaattcactc actgggaaca tacctgattg gctattggga agcaacagca ttgtgctatc   1200
   ttttaataat ttcacgagtg ggagctcagg tcaatgtcaa gggagcgtca atctagtgga   1260
   gagttattca cctgaaatga acagtttaaa taatgtccag ccatgcttga agaagaattt 1320
   cccatgtgct ttggatggac aatacagatc atccttgcat atcaattgtg gtgacaaaga 1380
   agcaatcatc aatggcacaa aatacgaagg tgacaccaca ccaaaaggtg cttccgtgtt 1440
   gtatgtaagc ccagactcaa actgggcatt cagcagcact gggaacttca tggacaacaa 1500
   catcaatgat gacaaataca ttgcgtcaag cacatcaaaa ctgacaatgc ccgactcaaa 1560
   gttgtatgca agagcacgcc tttctcctct ttcgctcaca tattacgggc gttgcatgca 1620
   taatgggagc tacacagtta aactccattt tgccgaaatt atattcacca atgacagtac 1680
   atactgcagc cttggcaaaa gaaaattcaa tgtgttcata cagggaagaa tggtgctaga 1740
   ggattttgat attgagcaat ctgctggtgg ggctggaaag ccagtcatca aggctttcaa 1800
   aacatatgtc acaaatcata cactgaagat tcaattctat tgggcaggaa gagggacaac 1860
   aggcattcca gatagagtat tttacggccc tctagtatct gcaatatcag taaatccaaa 1920
   cttccaaatt cctttggctg ttgaacctcc ccatactggc agtggcacaa aaacttcaag 1980
   gacagctaaa gctttgctga ttggagcccc aattattgcg atattcactg ctcttattgt 2040
   tggcatctac tggattaggc ggcgacggaa gaacttggtg aatcaagatc tccgggcact 2100
   tgacctccaa attggctcat ttaccttgag acaaatcaaa gcagcgacta ggaactttga 2160
   tccagcgaac aagattggcg aaggtggttt tggttcggtt tacaagggtt tgttgtccga 2220
   tggcaccatt attgctgtca aacagctatc atcgaagtcc aagcaaggga atcgtgaatt 2280
   tgtgaatgag ataggcatga tatctgcact ccagcatcca aaccttgtca ggttgtatgg 2340
   ctgttgtaca gaaggaaacc agctattgct agtttacgag tacatggaaa ataattgcct 2400
   tgcacgtgct ctatttgttg aagaatatag actggcattg gattggccaa caagacgtaa 2460
   gatttgcctg ggaatagcaa ggggtctggc atatatgcat gaggagtctg caataaggat 2520
   tgtgcaccga gatatcaagg ctagcaatat actgcttgac aaagatttgg atgctaagat 2580
   ctcagatttt ggtctagcaa agcttaatga agatggtcac acccacataa gcacgaaagt 2640
   agctggaact attggataca tggctcctga gtacgcaatg cgtggttatt tgacagacaa 2700
   agctgatgtt tacagttttg gggttgttgc tttggaaatt gtgagtggaa aaagcaacac 2760
   aaactacagg ccaaaggaag attttgttta tcttctcgat tgggcttgtg ttttacatga 2820
   gagaggaact ctactggagc tggtagatcc agacttagga tccaattact caacggaaga 2880
   ggcactcctg atgctgaacg tggctctctt atgcaccaac gcagcaccga ctcttagacc 2940
   aaagatgtcc aacgctgtga gccttcttga aggccatacc cccctgcaac ccttcctatc 3000
   agaactcagc cttgctgcaa acagcctgag ctcaagtggt ctacgcagaa acttctggga 3060
   aaatccaagt gagagccaga gcataacggc acaagcatca tataacaaca ccagtgactc 3120
   gtcatcttta gatgtagatg gtagcttgag acattctgcg acttaaatgt tagatgtatt 3180
   tcagatctgt acacctgtaa gaataactag ggctatgcca ctatgtctat tctggcagta 3240
   caatgtagct actaacataa aaacacctaa ttctact 3277
<210> 2
   <211> 1014
   <212> PRT
   <213> Hordeum
<220>
   <221> misc_feature
   <223> RNR8 protein sequence
<400> 2
   Met Gly Ala Arg Arg Thr Pro Cys Gly Trp Arg Leu Leu Phe Phe Leu
   1               5                   10                  15
   Ala Leu Leu Leu Ala Glu Val Arg His Gly Ser Ser Ser Ser Ser Ser
               20                  25                  30
   Gly Val Lys Ala Gly Ala Ala Ala Asp Ala Leu Pro Pro Arg Leu Ser
           35                  40                  45
   Ser Ala Glu Val His Thr Leu Arg Arg Ile Ala Ala Asn Met Gly Ile
       50                  55                  60
   Ser His Trp Asn Phe Ser Gly Asn Pro Cys Glu Pro Asn Gly Ser Leu
   65                  70                  75                  80
   Val Cys Asp Cys Ser Phe Asn Asn Asn Thr Ile Cys His Ala Thr Glu
                   85                  90                  95
   Ile Phe Leu Lys Glu Gln Asn Phe Thr Gly Gln Leu Pro Pro Asp Phe
               100                 105                 110
   Ala Asp Leu Pro His Leu Leu Gln Leu Asp Leu Ser Arg Asn Val Phe
           115                 120                 125
   His Gly Thr Val Pro Asp Arg Trp Ala Arg Met Arg Leu Gln Gly Leu
       130                 135                 140
   Ser Leu Met Gly Asn Arg Leu Ser Gly Pro Phe Pro Met Ala Leu Thr
   145                 150                 155                 160
   Arg Ile Thr Thr Leu Thr Ser Leu Ser Ile Glu Ala Asn Glu Phe Arg
                   165                 170                 175
   Gly Gln Ile Pro Ala Glu Ile Gly His Leu Thr Gln Ile Asp Lys Leu
               180                 185                 190
   Ile Ile Ser Thr Asn Glu Phe Thr Gly Pro Leu Pro Ala Ala Leu Ser
           195                 200                 205
   Leu Leu Thr Asn Leu Thr Asp Leu Arg Ile Ser Gly Asn Asn Leu Ser
       210                 215                 220
   Gly Arg Val Pro Asp Phe Leu Ala Lys Leu Thr Lys Leu Ala Lys Leu
   225                 230                 235                 240
   Gln Ile Glu Gly Ser Leu Leu Glu Gly Pro Ile Pro Leu Gly Leu Ser
                   245                 250                 255
   Lys Leu Thr Asn Leu Ser Asp Leu Arg Ile Ser Asp Leu Arg Gly Ser
               260                 265                 270
   Gly Ser Ala Phe Pro Asp Leu Ser Arg Met Pro Ser Met Lys Thr Leu
           275                 280                 285
   Val Leu Arg Asn Cys Ser Ile Ser Gly Gly Ile Pro Ser Tyr Ile Trp
       290                 295                 300
   Val Met Glu Asn Leu Lys His Leu Asp Leu Ser Phe Asn Glu Leu Thr
   305                 310                 315                 320
   Gly Lys Val Ser Asp Ser Ile Thr Leu Met Gly Ser Val Asp Tyr Ile
                   325                 330                 335
   Tyr Leu Thr Gly Asn Ser Leu Thr Gly Asn Ile Pro Asp Trp Leu Leu
               340                 345                 350
   Gly Ser Asn Ser Ile Val Leu Ser Phe Asn Asn Phe Thr Ser Gly Ser
           355                 360                 365
   Ser Gly Gln Cys Gln Gly Ser Val Asn Leu Val Glu Ser Tyr Ser Pro
       370                 375                 380
   Glu Met Asn Ser Leu Asn Asn Val Gln Pro Cys Leu Lys Lys Asn Phe
   385                 390                 395                 400
   Pro Cys Ala Leu Asp Gly Gln Tyr Arg Ser Ser Leu His Ile Asn Cys
                   405                 410                 415
   Gly Asp Lys Glu Ala Ile Ile Asn Gly Thr Lys Tyr Glu Gly Asp Thr
               420                 425                 430
   Thr Pro Lys Gly Ala Ser Val Leu Tyr Val Ser Pro Asp Ser Asn Trp
           435                 440                 445
   Ala Phe Ser Ser Thr Gly Asn Phe Met Asp Asn Asn Ile Asn Asp Asp
       450                 455                 460
   Lys Tyr Ile Ala Ser Ser Thr Ser Lys Leu Thr Met Pro Asp Ser Lys
   465                 470                 475                 480
   Leu Tyr Ala Arg Ala Arg Leu Ser Pro Leu Ser Leu Thr Tyr Tyr Gly
                   485                 490                 495
   Arg Cys Met His Asn Gly Ser Tyr Thr Val Lys Leu His Phe Ala Glu
               500                 505                 510
   Ile Ile Phe Thr Asn Asp Ser Thr Tyr Cys Ser Leu Gly Lys Arg Lys
   515 520 525
   Phe Asn Val Phe Ile Gln Gly Arg Met Val Leu Glu Asp Phe Asp Ile
   530 535 540
   Glu Gln Ser Ala Gly Gly Ala Gly Lys Pro Val Ile Lys Ala Phe Lys
   545 550 555 560
   Thr Tyr Val Thr Asn His Thr Leu Lys Ile Gln Phe Tyr Trp Ala Gly
   565 570 575
   Arg Gly Thr Thr Gly Ile Pro Asp Arg Val Phe Tyr Gly Pro Leu Val
   580 585 590
   Ser Ala Ile Ser Val Asn Pro Asn Phe Gln Ile Pro Leu Ala Val Glu
   595 600 605
   Pro Pro His Thr Gly Ser Gly Thr Lys Thr Ser Arg Thr Ala Lys Ala
   610 615 620
   Leu Leu Ile Gly Ala Pro Ile Ile Ala Ile Phe Thr Ala Leu Ile Val
   625 630 635 640
   Gly Ile Tyr Trp Ile Arg Arg Arg Arg Lys Asn Leu Val Asn Gln Asp
   645 650 655
   Leu Arg Ala Leu Asp Leu Gln Ile Gly Ser Phe Thr Leu Arg Gln Ile
   660 665 670
   Lys Ala Ala Thr Arg Asn Phe Asp Pro Ala Asn Lys Ile Gly Glu Gly
   675 680 685
   Gly Phe Gly Ser Val Tyr Lys Gly Leu Leu Ser Asp Gly Thr Ile Ile
   690 695 700
   Ala Val Lys Gln Leu Ser Ser Lys Ser Lys Gln Gly Asn Arg Glu Phe
   705 710 715 720
   Val Asn Glu Ile Gly Met Ile Ser Ala Leu Gln His Pro Asn Leu Val
   725 730 735
   Arg Leu Tyr Gly Cys Cys Thr Glu Gly Asn Gln Leu Leu Leu Val Tyr
   740 745 750
   Glu Tyr Met Glu Asn Asn Cys Leu Ala Arg Ala Leu Phe Val Glu Glu
   755 760 765
   Tyr Arg Leu Ala Leu Asp Trp Pro Thr Arg Arg Lys Ile Cys Leu Gly
   770 775 780
   Ile Ala Arg Gly Leu Ala Tyr Met His Glu Glu Ser Ala Ile Arg Ile
   785                 790                 795                 800
   Val His Arg Asp Ile Lys Ala Ser Asn Ile Leu Leu Asp Lys Asp Leu
                   805                 810                 815
   Asp Ala Lys Ile Ser Asp Phe Gly Leu Ala Lys Leu Asn Glu Asp Gly
               820                 825                 830
   His Thr His Ile Ser Thr Lys Val Ala Gly Thr Ile Gly Tyr Met Ala
           835                 840                 845
   Pro Glu Tyr Ala Met Arg Gly Tyr Leu Thr Asp Lys Ala Asp Val Tyr
       850                 855                 860
   Ser Phe Gly Val Val Ala Leu Glu Ile Val Ser Gly Lys Ser Asn Thr
   865                 870                 875                 880
   Asn Tyr Arg Pro Lys Glu Asp Phe Val Tyr Leu Leu Asp Trp Ala Cys
                   885                 890                 895
   Val Leu His Glu Arg Gly Thr Leu Leu Glu Leu Val Asp Pro Asp Leu
               900                 905                 910
   Gly Ser Asn Tyr Ser Thr Glu Glu Ala Leu Leu Met Leu Asn Val Ala
           915                 920                 925
   Leu Leu Cys Thr Asn Ala Ala Pro Thr Leu Arg Pro Lys Met Ser Asn
       930                 935                 940
   Ala Val Ser Leu Leu Glu Gly His Thr Pro Leu Gln Pro Phe Leu Ser
   945                 950                 955                 960
   Glu Leu Ser Leu Ala Ala Asn Ser Leu Ser Ser Ser Gly Leu Arg Arg
                   965                 970                 975
   Asn Phe Trp Glu Asn Pro Ser Glu Ser Gln Ser Ile Thr Ala Gln Ala
               980                 985                 990
   Ser Tyr Asn Asn Thr Ser Asp Ser  Ser Ser Leu Asp Val  Asp Gly Ser
           995                 1000                 1005
   Leu Arg  His Ser Ala Thr
       1010
<210> 3
   <211> 10626
   <212> DNA
   <213> Hordeum
<220>
   <221> misc_feature
   <223> RNR8 genomic sequence
<400> 3
   agaaagatcc tgtacttgga ctgcatagaa aggagccaag tcactccact catcctctcc     60
   taagagagac ggacctcgcc gctgccattg cgctccactt ggaacttgga gctggaggag    120
   agatgggggc gcggaggacg ccatgcggct ggaggctgct cttcttcctt gccttgcttc    180
   ttgccgaggt ccgccatggc tcctcttcat cttcttcagg ggttaaggcg ggagctgctg    240
   ctgatgccct ccctcctagg ctctcctctg ctgaaggtat gtgtgtgtgc tcagcacctg    300
   caaatgcatt gctctatctc tgccatcggg tgattcccct ctcctctgcg tttggctcaa    360
   tggttcggaa acatatatga ctctgataag cagagcgtga tttcttttgg cttccaaggc    420
   ttgatttttc tcctctttct ccttctttcc ccttttcgtc tcctttgctt ctcccatttt    480
   tggtcttctt tgcttggggc cgtcgaggag gagccagaac agtactgcaa aaagaacgaa    540
   ccaatgacaa aaaaaaactc tctaaacaag agagcaagat cgagaatctt ggatatattt    600
   tcttccaaag gaatgcaaac ccgccgcgtt catgtttttt ttttcctttt tgccattctc    660
   gtagtgcaca ctcttcgccg gattgcggcg aatatgggga tatcgcattg gaacttctcc    720
   ggcaatccct gtgaacccaa cggcagcttg gtgtgtgact gctccttcaa caacaatacc    780
   atatgccatg ccactgagat gtatgtatgc ataactcctt ggcgcttctc gtaaaacatt    840
   gggggaaacc tgtaaaattg atcttactac tagagtagaa ctgagcagat catgttcgtg    900
   catttctgtg ctctaacatg tgctacctgt tgcactgttc ttccgatcat ctttgcagat    960
   tcctcaagga acagaacttc accggccagc tcccaccaga ctttgctgat ctccctcatc   1020
   tcctccagct gtaagtacta aacagccagt caaactaatt taagccttgt tcagaaaaaa   1080
   taatctaact ttaggtgttt ctcaagataa tactaacacc ataattccgc attacaatgt   1140
   tgcagagacc taagcaggaa cgtgttccat ggcacagtgc ctgaccggtg ggcccggatg   1200
   aggctgcaag gactgtgagt ggcctcgccc ttcaccttac ccattgttat ctttgagtga   1260
   gtagtacagc aaaatgtagc atgtggagct attttggact ctatccaaga acaatatgcc   1320
   actaaaaaga tatatatttt gggcggaact ggtgaaggca cagtccctat ctatggagca   1380
   tgactgcaat gtcggcatgc caaagcatct cagaaggcaa caaattaggc tactcccaag   1440
   cacgattttc aaaatgccag gcatcttttg tgcagtgctc taccaacctt tttagcagtg   1500
   cagaatatag tctagcatga atccatgaag aataaaatca agcaataagc agcattttcc   1560
   gcgccgctgt cttgtcgatg cgatattaaa attgctacta tttctaacca ggccataggg   1620
   gagatgagat ctatgacaat gccctttaat tcatgacaaa ctagttacga ttttcttatg   1680
   agaagggcat tgcatcatta ccacttgacc gtgttggata tcgctaaccg gatgttcctt   1740
   ggatgggcaa acatggctgt agcaaataac tgcacccaga atgacagtag agctgcactg   1800
   cagaaaccag ggttgcaatg tattagtaaa aaaaatgttc gagacatatt taattgcaac   1860
   atattgctaa gaaaatttac aagtcatatt taagcctaaa tctgtgtgct tcgtttattg   1920
   gcttccagtc agaatactgt tgcaaaagag ttcaatttag agtgtccctt gcctgattgc   1980
   ctccttggag ctcagtctcc catgtctgga aagaggatat cattccggga gtgactaagc 2040
   actgtgtctg tgtggatgtt ctgccagcac gccctagatt ctagttgggg acctcagtaa 2100
   tgaactccca tttgtctcat ataggaagaa ctcgccatgt tttgttatca gaaaaatcta 2160
   ttgccatgtt ctcccatcgc aagaaaaaaa gaactcttca taaaacctta cattttccat 2220
   gtctgaagaa aaatacgagc acaagcttag aatgattcaa gttccagttt agttatactc 2280
   ccttcatttc atttacgaga catggtttag tatagcacgg aaattattcc ccaaaattat 2340
   actctctccg tttcataata taagtctttt tagagatttc actagaggac tacatacgga 2400
   tgtatataga catactttaa agtgtagatt cattcatttt actttgtatg tagtccctta 2460
   gtgaaatcgc ttaaaagact tatatttagg aacaaaggga gtattaaaaa agcttgttta 2520
   gtcataaacg ataaagggtt tcccctcgct ttatattata aagcaaccat tcgatacatc 2580
   cagcttgctg gagccgcagc acaaacaaac ccaaaaagaa aaaacgagac aaagaaagag 2640
   aaacaaatgc cgacaccgac agatcaatga agcaaaaatg gccggcaacc gctgcaccct 2700
   ccggagaagt tgcaccgcgc tcctagcact ctaaagcgtc gcatgacccg gcggcgccgg 2760
   caggcgtcgc cgcgttggag ccggacgagc cgccaaggat ttctctcgac ccaaaccccc 2820
   accaccacct cagatgatcc aaagtgcacc aaccaacttg ccgcccacca gcccgtgcca 2880
   ccacggtcac tgaaccaact ttgtcgtctt actgaggcca ccgacatgaa acctggagga 2940
   cagaagaaaa gacgatgggc tcgggggcat ccgtgacata gccgacacga ggggacatcc 3000
   tccaccgccg ccgcggagcc gatccgacgc agcgacaggg gcctacaggc actgatggct 3060
   cgaacggatc caaaagggtc cggatagccc ctgccgccac gctgcagctc gtcagccgac 3120
   gaagccgcca ccgctcgtag atcaccgcct cctcgccacc atcaccaggg agcagcgccg 3180
   ccgcgcgtcg agccgccagc ccgccccagc ccagaaagag cccgaaaagg cctagatctg 3240
   ggccgggcca ccagcaccac cacaccgtcc cacggccaac agccgcactg ccgcatcaag 3300
   ggcacccgag gcccgcggga accccgccac cgagccacac cgccaccacc atcgggaggc 3360
   tcccacgccc ctctacgcgt gcggggagga agaacggccc gtcgccgcca gcgccccgcg 3420
   ggcagggccc gacggaccat gccaccgccg gcgggagggg agaacgacgg ggagggtgct 3480
   ggaggagagg gggatcaggg ggttcgcccg tcgcccgcgg ggaagcgagc gagcgaacca 3540
   acgagaagtg gagttccggt ttttgtttag tcataaacga caagtaaatt gaggaacaga 3600
   gaaaaatcat tgaataacta gactggacaa gcatgcaata atgtgaaata ctcttatgaa 3660
   acaaatttct acttgttccc tgtgataaga atttcaatta tttccgcgtt ttaagccaca 3720
   aaattttgca taattgtaag aaatggaatg ccgtaaaaca gaattatgca gcgtgtcatt 3780
   gatctgttca tgccaaagtc ctgcaggtca ctaatgggaa acagattgtc agggcctttt 3840
   cccatggctc ttacgaggat cacaaccttg actagcctgt aagcaagaaa tttggtgaaa 3900
   acaactttga tttcctactt ttgagttctc ttttctaact acaatctgaa aataataatc 3960
   cgaaggagca ttgaagcaaa tgagttccgt gggcaaatcc cagcagaaat tgggcatctc 4020
   acgcaaatag ataagctgtg agtattttgc tttgcctaca ttgcttgtac aacagacaag 4080
   agttgcaggt atttggttaa catagttcct tatgacctat acactgatct ttgctcagga 4140
   taatatcaac caacgagttc actggacccc tgccggctgc tctttccttg ctgaccaatt 4200
   taaccgactt gtaaggattc tacgatcttt gtttttccct gtagccctct gcagactctt 4260
   tttgtaatgt tcggctattg aatctcctaa cagaaggatt tctggaaaca atttatctgg 4320
   aagggttcct gatttcttgg ctaaactgac aaagcttgca aaactgtaag ttggagagtt 4380
   catggcagaa cagattacaa atccacagat acccaagtat ataatttatt aaaggggatt 4440
   tctcacgatg caggcaaatc gaaggatctt tgctggaagg gcctattccc ctgggcttat 4500
   ccaaattgac aaacctttct gatctgtacg atgacaaatg tttgagatac tcaattctag 4560
   ctgcaccatc caatttattt attggattga agtgttaaac taaagcggga aattctggtt 4620
   gcgtctttct gttaaccagg aggattagtg atctgagagg cagtggatca gctttcccgg 4680
   atctaagcag aatgccatcg atgaaaacat tgtaagttat aatctgctga gttcccgtgc 4740
   tttttgtata taggttctca tataatatga actttttaaa tgtagagtcc tcaggaattg 4800
   ttcaatcagc ggaggcatcc cttcttacat atgggtcatg gaaaatctta agcatctgta 4860
   agttcatcgc attggttcca tatagactat agaacaatgc agtgtgttgg gagtacacat 4920
   aaaatatgtc agagctgaat gtactcggtg ttgacatgtt tttatcatca atgttctcta 4980
   tgcagggatc tgagctttaa tgaactgact ggaaaagtat cagattcgat cactcttatg 5040
   ggaagcgtag attacatgta agttggtatt tataataaat tgtactaggc atgctatttt 5100
   attgggctac tttcctgaat gcacctcaac aaaaacaatc ttgcagctat ctaactggaa 5160
   attcactcac tgggaacata cctgattggc tattgggaag caacagcatt gtgtaagttc 5220
   gtgtgtacat gcactctcca gagatgcatc attcttagat gctaatactt atagatattc 5280
   atgacttcct atcacatttt ttccttttcc cgaatctttt aaaagctgtc ataaaagtgc 5340
   agaacatatg agttaacata tctaagatat aaagtgttat taggtgtcaa tacccaattc 5400
   attcatgacg ggagaaaacc tataagatga tgttcttgat cacaagcatc cagagttagc 5460
   atttccttaa aaactattta ggcatgcctc tatctgctcc gtctgctaaa caggcatgcc 5520
   tctatctgct ctgtctatta aataattatg ttggctgcat aacagaatag ctcatgccca 5580
   tatgtacatg aaacttagag cttaagagaa tgtagtatga ctgctagtgc tatgattgaa 5640
   tctctaacag tctgaactaa aaaaaatgca tttcttactt gatgtactta aatttactat 5700
   gaaactatca aatgcaggga cctatctttt aataatttca cgagtgggag ctcaggtcaa 5760
   tgtcaaggga gcgtgtaaga aagctgtatc agaatttctg agtttttctc cgtgacaacg 5820
   attgtgcaaa taattaaata gtttcctatg attcttcaaa ttgcagcaat ctagtggaga 5880
   gttattcacc tgaaatgaac agtttgtaag attctttatc ctgttgcttc aaaagattaa 5940
   agagctttcg accaacagag aaatccagac ataataacct cttattttac tctcggtgat 6000
   tctacctttc tgtgcagaaa taatgtccag ccatgcttga agaagaattt cccatgtgct 6060
   ttggatggac aatgtaagtt gacgcccagt atttgttttg tcactgttgc ttctgatcac 6120
   cactgaatgg gaaaaacaaa ataacagatg tttgttgacg cttttcatac ccgtgctcat 6180
   gattgtgaca aacaagacta gctttaccaa tgaatcagaa gaagttacaa attgatattt 6240
   ctggattgta aaattatcag gataaactct aaaaaacaca agcaacaaga taactcacaa 6300
   ggatataatt agatcctgtg caaaagttca ttggtgctaa gcacacaagc caaacttttt 6360
   ttttcttgaa cacagaagcc aaacagaaaa gatcattgaa gctgtatgta atgatgttat 6420
   atgcattttc acagacagat catccttgca tatcaattgt ggtgacaaag aagcaatcat 6480
   caatggcaca aaatacgaag gtgacaccac accaaaaggt gcttccgtgt tgtatgtaag 6540
   cccagactca aactgggcat tcagcagcac tgggaacttc atggacaaca acatcaatga 6600
   tgacaaatac attgcgtcaa gcacatcaaa actgacaatg cccgactcaa agttgtatgc 6660
   aagagcacgc ctttctcctc tttcgctcac atattacggg cgttgcatgc ataatgggag 6720
   ctacacagtt aaactccatt ttgccgaaat tatattcacc aatgacagta catactgcag 6780
   ccttggcaaa agaaaattca atgtgttcat acaggtatga ccagataaga aaatattcgg 6840
   tcatttacat tttaataata gctgtatctt gttagctcct gtgatagtaa caagatactg 6900
   tatgatctca gggaagaatg gtgctagagg attttgatat tgagcaatct gctggtgggg 6960
   ctggaaagcc agtcatcaag gctttcaaaa catatgtcac aaatcataca ctgaagattc 7020
   aattctattg ggcaggaaga gggacaacag gcattccaga tagaggattt tacggccctc 7080
   tagtatctgc aatatcagta aatccaagta tgtcattttt aacacattat ttctgaattt 7140
   tgcataatgt gctccattaa tcactgtggg aagggtacat catttaacaa ggaacagcac 7200
   atgacggcat cttctgactt cttgcattac agttttgacg gagaaatact ctattcttta 7260
   atgatctggt gattttggtt tatgtttgtt catactcaga cttccaaatt cctttggctg 7320
   ttgaacctcc ccatactggc agtggcacaa aaacttcaag gacagctaaa gctttgctga 7380
   ttggagcccc aattattgcg atattcactg ctcttattgt tggcatctac tggattaggc 7440
   ggcgacggaa gaacttggtg aatcaaggtg ctataaatat tttttaatca actttttttt 7500
   ttatttatac ctccaatggc aaggacaggc ctggtgcagt ggtgaagtac tccccacttg 7560
   tgccaagagg tcctgggttc gacgtggcct ctctgcattg cactgtgcag gggtaaggct 7620
   tgcctcgtat aatccttccc cagatcccgc ctggtgtggg agcttctagc actgggtctg 7680
   tcctttacac cgtcaatgat aactggtgct tatctttcac tttgcttcac cgttcataca 7740
   caccatctat agcatccctc tactttccac ttctcagcag aacaagcaat agcaagatga 7800
   ctactatata gcaatgtaaa cctagtgaaa atcataatca tcaactatac gttctgaata 7860
   tttctgaccc atatgataac ttttattaga tctccgggca cttgacctcc aaattggctc 7920
   atttaccttg agacaaatca aagcagcgac taggaacttt gatccagcga acaagattgg 7980
   cgaaggtggt tttggttcgg tttacaaggt cagtcttcac tctctcccat gcttaaattc 8040
   tacaaactga agttaaatta catgattctt acaacgaaat aatgtttgtg ctagcagggt 8100
   ttgttgtccg atggcaccat tattgctgtc aaacagctat catcgaagtc caagcaaggg 8160
   aatcgtgaat ttgtgaatga gataggcatg atatctgcac tccagcatcc aaaccttgtc 8220
   aggttgtatg gctgttgtac agaaggaaac cagctattgc tagtttacga gtacatggaa 8280
   aataattgcc ttgcacgtgc tctatttggt aatcaatact tctatatttc ttggtgctcc 8340
   ccctaactag tattgcatgt taatcatgaa taatttgtat tgtgcggtcc agttgaagaa 8400
   tatagactgg cattggattg gccaacaaga cgtaagattt gcctgggaat agcaaggggt 8460
   ctggcatata tgcatgagga gtctgcaata aggattgtgc accgagatat caaggctagc 8520
   aatatactgc ttgacaaaga tttggatgct aagatctcag attttggtct agcaaagctt 8580
   aatgaagatg gtcacaccca cataagcacg aaagtagctg gaactatgta agtactttca 8640
   attatgtatt tttttccgaa atgcaaaatt ctatccacac attcccattt gatctatata 8700
   acaaaaagat aagaaggaaa agcagttaaa aggaaagtac tgcctctgta actttttata 8760
   ggacgttttt agagtcaatg acagtgtcaa aaaacgtctt atattaagtt acagagggag 8820
   taatagtgca gtgatgaaca atactccctc cgtctctatt tacatggcgt ccttgttttt 8880
   ttgtgatttc actttgaaca taatttggcc aattgataca ttaggaactt tttcgaatat 8940
   gagttcaatg gtatgatttt tatggcacat aaccaacggt tcgttggtca aattgatggt 9000
   caaggttgga tcttgaaatg tgtgggcatc atgtaaacag ggacggaggg agtataacat 9060
   ttacctccgg caaaaataca gaacatccat atagagataa aagttaacat caggtaagct 9120
   ggtcagaaac ccgcataaat gtagcacctt ggctttcctg catacaaaca taaattacaa 9180
   aaaaatgcaa tcaactttag attaacattt tccatgcaat gggatgctta aaaggtgttc 9240
   taattttcat tcaaacaact acggatggat attacttttc ggcatatctt tctaagtaat 9300
   tcctttagct gcgcagttta cagctgattt gattatttta tcattcttgc agtggataca 9360
   tggctcctga gtacgcaatg cgtggttatt tgacagacaa agctgatgtt tacagttttg 9420
   gggttgttgc tttggaaatt gtgagtggaa aaagcaacac aaactacagg ccaaaggaag 9480
   attttgttta tcttctcgat tgggtaactt ataaaccttc gtacccctct tgcagtaata 9540
   tggaccatac tgaatagact aatatatggt cacctcacat ccagagtaat taataagtgc 9600
   ctccgacaaa tatttgtgca aacaaagaac agttagacac gctgtcagtg cgatagggca 9660
   taaatcattg ttttatttaa catgtgtagc ataagtttac tacttaaacg gtaacattag 9720
   ctcaatcatg tctctcagcc ttccgtccct tacaaccaac cctctcctgg gcacatacaa 9780
   aatctaccca aatttctctt ggatatggat gaatttaatc taacctttaa ttaaaaattc 9840
   tggtaaataa tttggtcatc atgtggaggc atggatgcta gttgacatta tggatttttt 9900
   ttatgctttc agaatcagtt tgcgttaata tagtcataag aatcacaaat tgatctttgc 9960
   ctctagggaa acaagataag gatgtcattt gagagaatag ttacaggaaa ctgtatcatt 10020
   ttcatgtagg ggggtgggct gtagtgaaac aaaatgataa actctgacag ttcatgcttt 10080
   attctcgcat acataccttt aacgcttcga ggaagaggct gattgcatct cttaaatgtg 10140
   ttccaggctt gtgttttaca tgagagagga actctactgg agctggtaga tccagactta 10200
   ggatccaatt actcaacgga agaggcactc ctgatgctga acgtggctct cttatgcacc 10260
   aacgcagcac cgactcttag accaaagatg tccaacgctg tgagccttct tgaaggccat 10320
   acccccctgc aacccttcct atcagaactc agccttgctg caaacagcct gagctcaagt 10380
   ggtctacgca gaaacttctg ggaaaatcca agtgagagcc agagcataac ggcacaagca 10440
   tcatataaca acaccagtga ctcgtcatct ttagatgtag atggtagctt gagacattct 10500
   gcgacttaaa tgttagatgt atttcagatc tgtacacctg taagaataac tagggctatg 10560
   ccactatgtc tattctggca gtacaatgta gctactaaca taaaaacacc taattctact 10620
   aagaat 10626
<210> 4
   <211> 24
   <212> DNA
   <213> Artificial
<220>
   <223> Primer
<400> 4
   ctttcgtgct tatgtgggtg tgac 24
<210> 5
   <211> 22
   <212> DNA
   <213> Artificial
<220>
   <223> Primer
<400> 5
   catgaggagt ctgcaataag ga 22
<210> 6
   <211> 20
   <212> DNA
   <213> Artificial
<220>
   <223> oligonukleotid
<400> 6
   ggatttgtca cgtccaacct 20
<210> 7
   <211> 20
   <212> DNA
   <213> Artificial
<220>
   <223> oligonukleotid
<400> 7
   attggcaatt gtgatagccc 20
<210> 8
   <211> 24
   <212> DNA
   <213> Artificial
<220>
   <223> Primer NBS-LRR F1
<400> 8
   gctgaaccaa cccggggaga aata 24
<210> 9
   <211> 24
   <212> DNA
   <213> Artificial
<220>
   <223> Primer NBS-LRR F1
<400> 9
   agatgatcgg aagaacagtg caac 24